(19) Europäisches Patentamt
European Patent Office
Office européen des brevets

(11) **EP 3 517 080 A1**

(12) **EUROPÄISCHE PATENTANMELDUNG**

(43) Veröffentlichungstag:
**31.07.2019  Patentblatt 2019/31**

(51) Int Cl.:
*A61F 7/00* *(2006.01)*        *A61F 7/02* *(2006.01)*

(21) Anmeldenummer: **18154286.1**

(22) Anmeldetag: **30.01.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD TN**

(71) Anmelder: **Braincept AG**
**6203 Sempach Station (CH)**

(72) Erfinder: **Breiter, Michael**
**6280 Hochdorf (CH)**

(74) Vertreter: **Pollard, Peter Julian et al**
**c/o IP.DESIGN**
**Kanzlei und Patentbüro**
**Arbonerstrasse 35**
**8580 Amriswil (CH)**

(54) **WÄRMEAUFLAGE**

(57)     Wärmeauflage zur Abgabe von Wärmeenergie an den Körper einer Person. Die Wärmeauflage umfasst eine mit dem Körper der Person verbindbare Heizauflage. Die Heizauflage weist eine Oberseite und eine Unterseite auf und an deren Unterseite ist eine Klebefläche zum Ankleben der Heizauflage an der Hautoberfläche der Person vorgesehen. Die Heizauflage umfasst wenigstens ein Heizelement, durch das von einer elektrischen Energiequelle abgegebene elektrische Energie in Wärmeenergie wandelbar ist. Ferner umfasst die Wärmeauflage eine Steuereinheit, welche zur festen oder lösbaren Aufnahme der Energiequelle vorgesehen ist und die einen Signalverarbeiter aufweist, der zur Abgabe eines Steuersignals vorgesehen ist, mittels dessen die Energieabgabe von der Energiequelle an das Heizelement steuerbar ist. Die Heizauflage und die Steuereinheit sind durch wenigstens einen elektrischen Verbinder, über den die elektrische Energie zum Heizelement übertragbar ist, und wenigstens einen mechanischen Verbinder, elektrisch und mechanisch miteinander verbindbar.

Fig. 1

**Beschreibung**

Ausführungsbeispiele der vorliegenden Erfindung beziehen sich auf eine Wärmeauflage zur Abgabe von Wärmeenergie an den Körper einer Person. Weitere Ausführungsbeispiele beziehen sich auf ein System mit einer Wärmeauflage zur Abgabe von Wärmeenergie an den Körper einer Person und ein Verfahren zur Abgabe von Wärmeenergie an den Körper einer Person.

Rund 30% der Bevölkerung leidet an teils "chronischen" Nacken- und Rückenverspannungen. Chronische Nacken- und Rückenverspannungen können zu einer falschen Körperhaltung führen. Verspannungen und Fehlhaltungen können in einer schlecht durchbluteten Muskulatur resultieren und dies kann bspw. Migräne-Anfälle fördern. Des Weiteren wird oft bei einer Nacken- und Rückenverspannungen zur Vermeidung von Schmerzen eine Schonhaltung eigenommen. Eine andauernde Schonhaltung kann Gelenke enorm schädigen. Hinzu kommt, dass sich die Bandscheiben durch eine schlechte Körperhaltung verschleissen könne und so ein akuter Bandscheibenvorfall entstehen kann. Dies hat insbesondere langfristige folgen. Viele Betroffene versuchen die Verspannungen oder Schmerzen mit Hilfe von passiven Mitteln wie bspw. Salben oder Wärmepflaster zu beseitigen, ohne jegliche zusätzliche Anstrengungen oder Bewegung. Oftmals bleiben dabei Schmerzen bestehen und können chronisch werden.

Aus dem Stand der Technik sind zur Behandlung von Schmerzen, insbesondere bei Nacken- und Rückenverspannungen, wie vorhergehend erwähnt, beispielsweise Wärmepflaster bekannt. Diese können durch eine chemische Reaktion Wärme erzeugen. Diese Wärmepflaster können jedoch bei vielen Betroffen durch die chemische Reaktion, welche die Wärme erzeugt auch Hautausschläge verursachen. Ausserdem müssen Wärmepflaster mit chemischen Reaktionsstoffen regelmässig durch neue Wärmepflaster ersetzt werden. Dies ist nicht nachhaltig.

Des Weiteren kann durch ein Wärmepflaster nicht über einen längeren Zeitraum eine konstante Wärmer an der schmerzbetroffen bzw. verspannten Stelle erzeugt werden. Der Schmerz wird somit nur kurzfristig gelindert und insbesondere eine länger andauernde Behandlung ist teuer.

Ferner fehlt die Möglichkeit die Temperatur zu regulieren wodurch abhängig von der Person und dem Hauttyp Hautverletzungen wie bspw. Rötungen oder Verbrennungen bei zu viel Wärme entstehen kann.

Ferner kann die Wärmezufuhr nicht ein- und ausgeschaltet werden. Eine wechselnde Wärmezufuhr (ein- / ausschalten) ist mit herkömmlichen Wärmepflastern nicht möglich. Des Weiteren benötigen traditionelle Wärmepflaster ungefähr 30 Minuten, bis die therapeutische Wärme von ca. 40° C erreicht wird. Eine andauernde Wärmezufuhr kann je nach Gebrechen einen negativen Einfluss auf den Heilungsprozess haben. Eine andauernde Wärmezufuhr kann zu einer Gewöhnung des Muskelgewebes führen, wodurch ein entspannender Effekt ausbleibt. Es besteht somit nicht die Möglichkeit von intelligenten Wärme-Therapie-Verfahren, da die Wärme konstant abgegeben wird.

Es besteht auch die Gefahr, einer falschen Anwendung von Wärme-Therapie. Oftmals werden Wärmepflaster nur an der Stelle aufgetragen, wo der Schmerz vorkommt bzw. lokalisiert wurde. Dies kann den Schmerz und die Verspannung kurzfristig lösen, jedoch können an anderen Körperstellen neue Verspannungen entstehen, da sich die wärmende Stelle entlastet, während sich andere Stellen dadurch verkrampfen. Dadurch entstehen weitere Verspannungen aufgrund falscher Anwendung von lokaler Wärme.

Weiter sind auch grössere Heizkissen bekannt, welche direkt über eine Steckdose am Strom angeschlossen werden können. Diese sind relativ unflexibel und schränken, aufgrund des Netzkabels welches mit einer Steckdose verbunden sein muss, im Alltag die Bewegungsfreiheit stark ein.

Aufgabe der vorliegenden Erfindung ist es, ein verbessertes Konzept zur Abgabe von Wärmeenergie an den Körper einer Person bereitzustellen, dass kostengünstiger und nachhaltiger ist.

Diese Aufgabe wird durch eine Vorrichtung nach Anspruch 1 ein Verfahren nach Anspruch 14, und ein Computerprogramm gemäss Anspruch 16 gelöst.

Ausführungsbeispiele der vorliegenden Erfindung schaffen eine Wärmeauflage zur Abgabe von Wärmeenergie an den Körper einer Person. Die Wärmeauflage umfasst eine mit dem Körper der Person verbindbare Heizauflage. Die Heizauflage weist eine Oberseite und eine Unterseite auf und an deren Unterseite, ist eine Klebefläche zum Ankleben der Heizauflage an der Hautoberfläche der Person vorgesehen. Die Heizauflage umfasst wenigstens ein Heizelement, durch das von einer elektrischen Energiequelle abgegebene elektrische Energie in Wärmeenergie wandelbar ist. Ferner umfasst die Wärmeauflage eine Steuereinheit, welche zur festen oder lösbaren Aufnahme der Energiequelle vorgesehen ist und die einen Signalverarbeiter aufweist, der zur Abgabe eines Steuersignals vorgesehen ist, mittels dessen die Energieabgabe von der Energiequelle an das Heizelement steuerbar ist. Die Heizauflage und die Steuereinheit sind durch wenigstens einen elektrischen Verbinder, über den die elektrische Energie zum Heizelement übertragbar ist, und wenigstens einen mechanischen Verbinder, elektrisch und mechanisch miteinander verbindbar.

Ausführungsformen stellen Konzepte bereit, um Wärmeenergie an den Körper einer Person abzugeben. Dabei kann die Heizauflage durch eine Klebefläche an den Körper einer Person angeordnet bzw. angeklebt oder befestigt werden. Die Heizauflage kann eine mehr oder weniger flache Form aufweisen bzw. flachförmig ausgebildet sein, sodass möglichst keine Druckstellen am Körper der Person beim Tragen der Wärmeauflage entstehen. Ferner ist die Heizauflage durch eine möglichst flache Form flexibel. Die Heizauflage kann eine im wesentliche flache Form mit einer Unterseite bzw. einer ersten Fläche und einer Oberseite bzw. einer zweiten Fläche aufweisen. Die Klebefläche kann an der Unterseite der Heizauflage vorgesehen oder ausgebildet sein. Die Klebefläche kann mit einer Schutzschicht gegen unbeabsichtigtes Ankleben beaufschlagt sein, welche vor dem ankleben an den Körper entfernt werden kann. Die Heizauflage kann elektrische Energie bzw. elektrischen Strom aufnehmen und bspw. in Wärmeenergie bzw. thermische Energie umwandeln. Dies Umwandlung kann beispielsweise durch einen elektrische Widerstand oder ein elektrisches Wärme- oder Heizelement erfolgen. Der elektrische Widerstand bzw. das elektrische Heizelement kann elektrische Energie in Wärme umwandeln. Die Wärme kann durch den Kontakt der Heizauflage mit dem Körper der Person, an den Körper der Person abgegeben werden.

Der Muskel oder die Muskeln welcher sich unter der Haut an der Stelle der Heizauflage befinden, welche Wärme an den Körper abgibt, kann durch die Wärme, welche von der Heizauflage an den Körper der Person abgegeben wird, stimuliert werden. Durch die Abgabe von Wärme an Muskeln können sich zusammengezogene bzw. kontraktierte Muskeln entspannen. Ferner kann durch die Wärmeabgabe an Muskeln die Durchblutung des Muskels gefördert werden.

Durch die Stimulierung der Muskeln können sich Verkrampfungen lösen und Schmerzen können reduziert werden. Es ist auch möglich, dass der Antagonist eines Muskels oder einer Muskelgruppe stimuliert wird, um den oder die verkrampften Gegenspieler zu entspannen. Durch die entspannten Muskeln und die entspannte Muskulatur im Allgemeinen, verbessert sich die Körperhaltung und/oder Bewegung der Person. Mit Körperhaltung, welche eher statisch verstanden werden kann, ist im Folgenden auch immer eine Körperbewegung, welche eher dynamisch verstanden werden kann gemeint. Durch eine gute Körperhaltung können Gelenke geschont und Schmerzen reduziert werden. Dadurch kann das Wohlbefinden der Person gesteigert werden.

Durch die Verwendung eines elektrischen Energiespeichers anstelle eines chemischen Energiespeichers, wie bspw. bei einem Wärmepflaster, besteht keine Gefahr allergischer Hautreaktionen gegenüber dem chemischen Energiespeicher.

Die Wärmeauflage kann ferner die Steuereinheit umfassen, an welchem ein Signalverarbeiter, Prozessoreinheit oder Controller angeordnet sein kann. Der Signalverarbeiter kann ausgebildet sein, ein Steuersignal bzw. Signal zur Steuerung einer elektrischen Energiequelle zu erzeugen. Der Signalverarbeiter kann Daten bzw. Signale empfangen und verarbeiten und Daten bzw. Signale ausgeben. Die Signale können als Funksignale oder leitungsgebundene Signale, Analog- oder Digitalsignale empfangen oder ausgegeben werden. Die Daten können bspw. von Sensoren oder einer Steuerung erzeugt und dem Signalverarbeiter zugeführt werden. Die Verarbeitung kann bspw. durch Auswerten oder Bewerten bzw. Vergleichen der gemessenen bzw. erfassten Daten mit Referenzdaten erfolgen. Die Verarbeitung kann Software oder Hardwaremässig erfolgen.

Der Signalverarbeiter kann ausgebildet sein den Energiefluss einer elektrischen Energiequelle zu steuern. Der Signalverarbeiter kann ausgebildet sein, die thermische Energiezufuhr oder Energieabgabe an den Körper bzw. die Muskulatur zu steuern. Die elektrische Energie kann bspw. kontinuierlich durch Leistungsanpassung oder durch verändern eines Puls-paus-verhältnisses, von der elektrischen Energiequelle an die Heizauflage abgegeben werden.

Die elektrische Energiequelle kann z.B. eine Batterie oder einem Akku sein. Die Batterie bzw. der Akku kann elektrische Energie bzw. elektrischen Strom abgeben. Ein Akku kann über eine weitere Energiequelle und eine Ladeschaltung mit elektrischer Energie aufgeladen oder nachgeladen werden.

Somit kann beispielsweise ein Akku oder eine Batterie als mobile elektrische Energiequelle die elektrische Energie für die Heizauflage zur Verfügung stellen. Zusätzlich kann die elektrische Energiequelle den Signalverarbeiter, Sensoren, Funkeinheit, Vibrationserzeuger, Elektroden, Heizelement, oder Übertragungseinheiten mit elektrischer Energie versorgen. Die Batterie oder der Akku kann an der Steuereinheit befestigt oder befestigbar sein. Die elektrische Energie kann aber auch an die Steuereinheit bspw. über ein Kabel übertragen werden. Das Kabel kann mit einem Netzteil bzw. einer Steckdose verbunden sein, welche die Energie bereitstellt. Somit kann die Energiequelle, welche lösbar in der Steuereinheit aufnehmbar ist auch eine externe Energiequelle sein, welche über ein Kabel bspw. ein Netzkabel in der Steuereinheit aufgenommen ist.

Über einen elektrischen Verbinder bzw. ein elektrisches Verbindungselement kann der elektrische Strom von der Steuereinheit oder der elektrischen Energiequelle auf die Heizauflage übertragen werden. Durch den elektrischen Verbinder kann eine elektrische Kopplung und eine Trennung zwischen der Steuereinheit bzw. der elektrischen Energiequelle und der Heizauflage ermöglicht werden. Durch komplementäre elektrische Verbindungselemente wie beispielsweise ein Stecker bzw. Stift und eine dazu passende Buchse oder Magnete oder Druckknöpfe, kann eine sichere und zuverlässige elektrische Verbindung hergestellt werden, die getrennt werden kann. Komplementäre Verbindungselemente sind Verbindungselemente, welche eine kraft- und/oder formschlüssige Verbindung zueinander herstellen.

Zusätzlich kann mindestens ein mechanischer Verbinder bzw. ein mechanisches Verbindungselement an der Steuereinheit und ein Komplementäres mechanisches Verbindungselement an der Heizauflage angeordnet werden. Das Verbindungselement kann an der Oberseite der Heizauflage bzw. an der Klebefläche gegenüberliegenden Fläche der Heizauflage angeordnet sein. Dadurch kann die Heizauflage zwischen der Steuereinheit und dem Körper der Person angeordnet werden. Durch den mechanischen Verbinder kann eine sichere und zuverlässige mechanische Kopplung zwischen der Steuereinheit und der Heizauflage hergestellt werden, welche getrennt werden kann. Durch komplementäre mechanische Verbindungselemente wie beispielsweise vorhergehend bei den elektrischen Verbindungselementen erwähnt, kann eine sichere und zuverlässige mechanische Verbindung hergestellt werden, welche getrennt werden kann. Durch die mechanischen Verbindungselemente kann die Heizauflage nach Gebrauch zur Reinigung oder Entsorgung von der Steuereinheit getrennt werden. Die Steuereinheit kann beliebig oft wiederverwendet werden, wodurch die Unterhalts- oder Betriebskosten der Wärmeauflage gesenkt werden können und ein nachhaltiger Nutzen entsteht.

Bei einer bevorzugten Ausgestaltung weist der elektrische Verbinder ein mit der Steuereinheit verbundenes erstes elektrisches Verbindungselement und ein mit der Heizauflage verbundenes zweites Verbindungselement auf. Der mechanische Verbinder weist ein mit der Steuereinheit verbundenes erstes mechanisches Verbindungselement und ein mit der Heizauflage verbundenes zweites mechanisches Verbindungselement auf. Dabei umschliesst das erste und zweite mechanische Verbindungselement das dazu korrespondierende erste bzw. zweite elektrische Verbindungselement. Der elektrische Verbinder oder das elektrische Verbindungselement bzw. die elektrische Schnittstelle kann in dem mechanischen Verbinder oder dem mechanische Verbindungselement angeordnet bzw. integriert sein. Der mechanische Verbinder oder das mechanische Verbindungselement kann auch in dem elektrischen Verbinder oder dem elektrische Verbindungselement angeordnet bzw. integriert sein. Das bietet den Vorteil, dass weniger Verbindungselemente beim Verbinden der Heizauflage mit der Steuereinheit miteinander verbunden bzw. gekoppelt werden müssen. Dadurch wird die Bedienung bzw. das Handling vereinfacht. Als Verbindungselement können bspw. metallische Druckknöpfe, Magnete oder ein Klicksystem verwendet werden. Das Verbindungssystem sollte zum Einen eine mechanisch stabile, lösbare Verbindung herstellen, zum Anderen sollte ein möglichst kleiner elektrischer Widerstand auftreten.

Bei einer besonders bevorzugten Ausgestaltung sind an der Steuereinheit mindestens drei mechanische Verbindungselemente und gegenüberliegend an der Heizauflage an gleicher Stelle, drei komplementäre mechanische Verbindungselemente ausgebildet. Durch drei mechanische Verbindungselemente kann eine stabile mechanische Verbindung zwischen der Heizauflage und der Steuereinheit hergestellt werden. Dies insbesondere, wenn die Wärmeauflage am Körper einer Person befestigt wird. Durch mehr als zwei mechanische Verbindungelemente ist beim Befestigen der Wärmeauflage am Körper der Person weniger auf die Position der Verbindungselemente zu achten. Ausserdem kann mit drei oder mehr Verbindungselementen, insbesondere bei einer asymmetrischen Anordnung der Verbindungselemente, einen positionsoptimierte bzw. positionsbestimmte Verbindung zwischen Heizauflage und Steuereinheit vorgegeben werden. Dies bedeutet, dass die Steuereinheit nur in einer Position mit der Heizauflage verbindbar ist. Somit können bspw. falsche elektrische Verbindungen und damit Fehlfunktionen oder Kurzschlüsse zwischen der Steuereinheit und der Heizauflage verhindert werden.

Bei einer bevorzugten Ausgestaltung ist die Energiequelle und das Heizelement ausgebildet die Heizauflage in weniger als 1 min, vorzugsweise in weniger als 20 sek., besonders bevorzugt in weniger als 5 sek. um 20° C zu erwärmen. Das bietet den Vorteil, dass dem Körper z.B. an Trigger Punkten der Muskulatur durch die schnelle Erwärmung, schnell Wärmeenergie zugeführt werden kann. Dies kann eine besonders wirkungsvolle Stimulation der Muskulatur und dadurch eine Entspannung der Muskulatur bewirken. Durch eine Erwärmung um 20°C kann die Heizauflage schnell von Raumtemperatur auf 38° bis 45° C (therapeutische Wärme) erwärmt werden. Wobei der Körper im Bereich der eigenen Körpertemperatur (36°C) sehr sensibel auf Wärme reagiert und Temperaturänderungen in diesem Bereich deutlich wahrgenommen werden.

Bei einer besonders bevorzugten Ausgestaltung umfasst die Wärmeauflage mindestens einen Körperhaltungssensor. Der Körperhaltungssensor kann ausgebildet sein, die Körperhaltung und/oder Bewegung der Person zumindest in Teilen zu erfassen. Der Körperhaltungssensor kann Körperhaltungsdaten erzeugen und der Signalverarbeiter kann ausgebildet sein, abhängig von den Körperhaltungsdaten das Steuersignal zur Steuerung der elektrischen Energiequelle zu erzeugen bzw. mittels der Körperhaltungsdaten die Energieabgabe von der Energiequelle an das Heizelement zu steuern. Der Körperhaltungssensor kann an der Steuereinheit angeordnet bzw. ausgebildet sein. Der Körperhaltungssensoren kann mindestens einen der folgenden Sensoren bspw. ein 3D-Gyro-Sensor, ein Beschleunigungssensor, ein Biegesensor, ein Temperatursensor oder ein Sensor mit Dehnmessstreifen umfassen. Durch die Körperhaltungsdaten, welche der oder die Körperhaltungssensoren ausgibt, kann die Körperhaltung bzw. die Bewegung bestimmt werden. Die Körperhaltungsdaten können dem Signalverarbeiter zugeführt bzw. übermittelt werden.

Durch einen Körperhaltungssensor bzw. durch die erzeugten Körperhaltungsdaten kann erkannt bzw. festgestellt werden, ob die Körperhaltung der Person korrekt ist oder ob die Körperhaltung falsch bzw. schlecht ist. Es ist auch möglich, nur einem Teil der Körperhaltung z.B. die Haltung des Oberkörpers einer Person, mit mindestens einem Körperhaltungssensor zu erfassen und Körperhaltungsdaten zu erzeugen.

Eine korrekte Körperhaltung kann eine entspannte Muskulatur umfassen. Bei einer korrekten Körperhaltungen kann der gesamte Körper aufgerichtet sein. Bei einer korrekten Körperhaltungen sollte der Körper sich nicht gegen die linke oder rechte Körperseite neigen. Der Kopf bzw. der Körper kann bei einer korrekten Körperhaltung erscheinen, wie wenn er an einem unsichtbaren Faden, welcher am höchsten Punkt des Kopfes austritt befestigt bzw. daran aufgehängt wäre. Bei einer falschen bzw. schlechten Körperhaltung kann sich der Körper zu weit nach vorne, hinten, links oder rechts neigen. Es ist auch möglich, dass z.B. die Schultern zu stark nach oben oder unten gezogen sind. Ferner kann ein Buckel oder ein Hohlkreuz eine schlechte oder falsche Körperhaltung sein. Eine korrekte Körperhaltung ist ein Hinweis für eine entspannte Muskulatur. Eine falsche Körperhaltung ist ein Hinweis auf eine verspannte oder verkrampfte Muskulatur.

Eine entspannte Körperhaltung kann personenabhängig sein. Gewisse Personen können bspw. angeborene oder durch Krankheit, Unfall oder Alter veränderte Körperhaltungen aufweisen. Es ist auch möglich dies veränderte Körperhaltung als korrekte Körperhaltung zu definieren und Abweichungen von dieser veränderten Körperhaltung als Körperfehlhaltung oder als falsche Körperhaltung zu erfassen. Es kann somit bspw. auch eine Kalibrierung der aktuellen Körperhaltung oder korrekten Körperhaltung möglich sein, damit Personen bspw. mit angeborenen Haltungsstörungen keine verfälschte Körperhaltungsdaten aufweisen.

Die Bestimmung der Körperhaltung kann durch auswerten oder bewerten der Körperhaltungsdaten bzw. vergleichen der gemessenen bzw. erfassten Körperhaltungsdaten mit Referenzkörperhaltungsdaten in dem Signalverarbeiter erfolgen. Durch die Bestimmung der Körperhaltung kann erkannt werden, ob beispielsweise Verspannungen im Körper bzw. in der Muskulatur der Person vorhanden sind. Verspannungen der Muskulatur können Schmerzen an verschiedenen Körperstellen wie beispielsweise im Nacken oder im Rücken verursachen. Die Körperhaltungsdaten können exakt zeigen, wo das Problem bekämpft bzw. gelöst werden muss.

Wird aufgrund der Körperhaltung erkannt, dass ein bestimmter oder mehrere Muskel verspannt sind, können diese beispielsweise durch Zufuhr von Energie bzw. Wärme stimuliert bzw. entspannt werden. Der Signalverarbeiter kann abhängig von den Körperdaten das Signal mittels dessen die Energieabgabe von der Energiequelle an das Heizelement steuerbar ist verändern.

Bei einer weiteren bevorzugten Ausgestaltung umfasst die Wärmeauflage eine Funkeinheit zum Senden und/oder Empfangen von Daten. Die Daten können Sensordaten, Steuerdaten, Statusdaten oder andere Daten umfassen. Die Funkeinheit kann auf der Steuereinheit angeordnet oder ausgebildet sein. Durch die Funkeinheit kann eine erste Wärmeauflage mit mindestens einer zweiten Wärmeauflage Daten austauschen. Durch die Funkeinheit kann die Wärmeauflage aber auch Daten mit einem Mobilgerät wie bspw. einem Tablet, einem Mobiltelefon oder einem Smartphone austauschen. Die Daten können zwischen dem Signalverarbeiter auf der ersten Wärmeauflage und einem Signalverarbeiter auf der zweiten Wärmeauflage oder einem Signalverarbeiter auf einem Mobilgerät ausgetauscht werden. Damit kann z.B. die Benutzerfreundlichkeit bei der Steuerung und die Effizienz bei der Behandlung erhöht werden.

Bei einer bevorzugten Ausgestaltung umfasst die Wärmeauflage einen Temperatursensor, welcher ausgebildet ist Temperaturdaten zu erzeugen und der Signalverarbeiter ist ausgebildet, abhängig von den Temperaturdaten das Steuersignal zur Steuerung der elektrischen Energiequelle zu erzeugen. Der Temperatursensor kann an der Wärmeauflage bspw. an der Heizauflage angeordnet sein. Der Temperatursensor kann die Oberflächentemperatur der Haut und/oder die Temperatur der Heizauflage erfassen bzw. messen. Es können auch mehrere Temperatursensoren an der Wärmeauflage bzw. der Heizauflage angeordnet sein. Abhängig von der erfassten Temperatur des Temperatursensors kann das Steuersignal zur Steuerung der elektrischen Energiequelle bzw. des elektrischen Stroms erzeugt werden. Bspw. der Signalverarbeiter kann ausgebildet sein, das Steuersignal zur Steuerung des elektrischen Stroms abhängig von den Temperaturdaten bzw. der erfassten Temperatur zu erzeugen und damit die Abgabe der Wärmeenergie bzw. die Temperatur der Heizauflage zu steuern bzw. zu regeln. Somit kann die Temperatur der Heizauflage gesteuert oder geregelt werden.

Durch das Messen der Körpertemperatur bzw. der Oberflächentemperatur der Haut kann der Person bspw. ein angenehmes, warmes Körpergefühl vermittelt werden. Ferner kann die Temperatur der Heizauflage auf die optimale Behandlungstemperatur abgestimmt werden. Ferner kann verhindert werden, dass es durch zu starkes Aufwärmen der Heizauflage zu Verbrennungen oder anderen Hautverletzungen kommt.

Abhängig von der Person und dem Hauttyp können unterschiedlich hohe Temperaturen an der Heizauflage als angenehm wahrgenommen werden und eine Stimulierung bzw. eine Heilung des Muskels bewirkten. Wenn die Heizauflage zu schwach erwärmt wird, wird der Muskel nicht stimuliert bzw. nicht entspannt und somit besteht keine positive Wirkung für die Person, da bspw. der Schmerz nicht gelindert wird. Durch das Erfassen bzw. Messen der Temperatur der Heizauflage und vergleichen mit einer Sollwert bzw. einer Solltemperatur kann eine optimale Temperatur an der Heizauflage erzeugt werden. Misst der Temperatursensor auf der Haut eine Temperatur, welche kleiner ist als eine Solltemperatur, so kann die Heizauflage bspw. durch den Signalverarbeiter aktiviert werden und elektrische Energie in Wärmeenergie umwandeln. Durch die Abgabe der Wärmeenergie an die Haut, kann die Haut bis zu einer vorgegebenen Solltemperatur erwärmt werden. Nach Erreichen der Solltemperatur wird die Umwandlung der Wärmeenergie in der Heizauflage reduziert oder eingestellt, bis die Temperatur der Haut erneut unter einen vorgegebenen Wert abfällt.

Die Temperatur wird vorzugsweise an der auf der Haut aufliegenden Fläche (Klebefläche) der Heizauflage erfasst. Der elektrische Strom wird abhängig von der Temperatur der Heizauflage entsprechend geregelt. Falls die Solltemperatur der Heizauflage höher sein soll als die gemessene Temperatur kann der elektrische Strom bzw. die Abgabe der elektrischen Energie an die Heizauflage erhöht werden. Falls die Solltemperatur der Heizauflage tiefer sein soll als die gemessene Temperatur, kann der elektrische Strom bzw. die Abgabe der elektrischen Energie zu der Heizauflage reduziert werden. Somit kann eine vorgegebene bzw. optimale Temperatur an der Klebefläche der Heizauflage erzielt werden. Die Wärmeabgabe kann dabei an der gesamten Fläche bzw. Klebefläche oder Folienfläche der Heizauflage konstant sein.

Es hat sich bei Tests gezeigt, dass die Temperatur der Heizauflage beim Kontakt mit der Haut < 60 °C, vorzugsweise < 50°C, besonders bevorzugt < 45°C sein sollte, um Hautverletzungen zu vermeiden. Insbesondere bspw. bei kleinen Kindern sollte die Temperatur zur Vermeidung von Hautverletzungen < 40°C sein. Um eine Stimulation des Muskels zu erreichen sollte die Temperatur > 35°C, vorzugsweise > 40°C, besonders bevorzugt > 45°C sein.

Ferner kann das Wohlbefinden bezüglich der Temperatur einer Heizauflage von deren Grösse abhängen. Dabei gilt, je grösser die Heizauflage desto tiefer sollte die Temperatur sein, bei welcher diese betrieben wird, da bei grossen Heizauflagen die Gefahr von Hautverletzungen grösser ist. Zusätzlich besteht die Gefahr, dass der Körper der Person bei grossflächigen Heizauflagen überhitzt.

Bei einer weiteren bevorzugten Ausgestaltung sind an der Unterseite der Heizauflage Elektroden angeordnet und der Signalverarbeiter ist ausgebildet durch Elektromyografie die Muskelspannung und/oder durch messen des Leitwertes der Haut den Hautwiderstand zu bestimmen. Die Wärmeauflage kann Elektromyografie-Elektroden zur Messung der Muskelspannung umfassen. Die Elektroden zur Messung der Muskelspannung EMG können an der Heizauflage angeordnet sein. Durch Elektromyografie (EMG) kann die elektrische Muskelaktivität gemessen werden. Mit Elektroden welche an der Hautoberfläche angeordnet werden, lassen sich Potentialänderungen eines Muskels oder mehrerer Muskeln messen. Die Messung der Potentialänderungen mit einem EMG, gibt Informationen über die Muskelspannung. Die Information der Muskelspannung kann dem Signalverarbeiter zur Verfügung gestellt werden. Der Signalverarbeiter kann ausgebildet sein abhängig von der Muskelspannung abgestimmtes Steuersignal zur Steuerung der elektrischen Energiequelle zu erzeugt. Durch die Information der Muskelspannung kann eine gezielte Entspannung durch die Wärmezufuhr an die Muskel erreicht werden. Somit lassen sich Verspannungen in der Muskulatur feststellen und beheben. EMG-Sensoren können auch Hautdaten identifizieren bzw. erfassen. Weitere Sensoren zur Erfassung der Muskelspannung können Körperhaltungssensoren wie ein 3D-Gyro-Sensor (Gyroskop 3D), ein Beschleunigungssensor, ein Temperatursensor, ein Biegesensor oder ein Dehnmessstreifen sein.

Die Wärmeauflage kann Elektroden zum erfassen des Hautwiderstands umfassen. Die Wärmeauflage kann einen Hautwiderstandssensor umfassen, welcher ausgebildet ist den Hautwiderstand der Person zu erfassen. Der Signalverarbeiter kann ausgebildet sein abhängig vom Hautwiderstand abgestimmtes Steuersignal zur Steuerung der elektrischen Energiequelle bzw. zur Steuerung der Energieabgabe von der Energiequelle an das Heizelement, zu erzeugen. Durch das Erfassen des Hautwiderstandes kann bspw. festgestellt werden, wie stark die Person schwitzt. Die Wärmeauflage kann auch einen Sensor zur Erfassung der Feuchtigkeit bzw. der Hautfeuchtigkeit umfassen. Solche Feuchtigkeitssensoren verändern bspw. ihren elektrischen Wiederstand in Abhängigkeit zur Feuchtigkeit. Die Abgabe von elektrischer Energie durch die elektrische Energiequelle und damit die Wärmeabgabe von der Wärmeauflage, kann damit in Abhängigkeit des Hautwiderstandes oder von der Intensität der Schweissabgabe bzw. des Transpirierens einer Person reguliert bzw. verändert werden.

Zur Bestimmung des Hautwiderstands kann auch die Impedanz zwischen zwei Elektroden, welche am Körper anliegen, gemessen werden. Die Impedanz ist ebenfalls vom Schweiss an der Hautoberfläche abhängig. Der Signalverarbeiter kann ausgebildet sein die Impedanz zu bestimmen bzw. zu messen. Der Signalverarbeiter kann ausgebildet sein, ein auf die Impedanz abgestimmtes Steuersignal zur Steuerung der Energieabgabe von der Energiequelle an das Heizelement zu erzeugen.

Bei einer weiteren bevorzugten Ausgestaltung umfasst die Klebefläche der Heizauflage einen Klebstoff, vorzugsweise einen Haftklebstoff. Ein Haftklebstoff bietet den Vorteil, dass der Klebstoff keinen Verfestigungsmechanismus aufweist, wodurch der Klebstoff keinen dauerhafte Verbindung eingeht. Somit lässt sich die Heizauflage nach Gebrauch von der Körperoberfläche bzw. Hautoberfläche des Benutzers lösen und bei Bedarf wieder an den Körper einer Person ankleben.

Bei einer bevorzugten Ausgestaltung ist der Klebstoff gelförmig ausgebildet. Der Klebstoff an der Klebefläche der Heizauflage kann einen gelförmigen Klebstoff umfassen bzw. die Konsistenz des Klebstoffs kann gelförmig sein. Gelförmige Substanzen können sich gut unebenen Oberflächen wie z.B. der Hautoberfläche anpassen und dies grossflächig kontaktieren. Durch die Kontaktierung grosser Flächen, lässt sich die Wärmeenergie gut von der Heizauflage auf den Körper der Person übertragen.

Bei einer weiteren bevorzugten Ausgestaltung ist die Heizauflage ein Wegwerfelement und die Steuereinheit ist ein wiederverwendbares Element. Die Heizauflage kann auf den Körper bzw. die Haut der Person aufgeklebt werden. Aus hygienischen Gründen (Schweiss, Körperfett) oder z.B. weil verschieden Personen die Wärmeauflage nutzen oder weil nach einer gewissen Zeit die Klebewirkung des Klebstoffes nachlässt, kann die Heizauflage nach einer gewissen zeitlichen Benutzungsdauer oder nach einer gewissen Anzahl Benutzungen z.B. nach ca. 30 Anwendungen entsorgt werden.

Die Steuereinheit kann die Elektronik bzw. verschiedene elektronische Elemente umfassen, welche beliebig wiederverwendet werden können. Die Heizauflage kann nach Abnutzung von der Steuereinheit entfernt werden und durch eine neue Heizauflage ersetzt werden. Durch ein Wiederverwenden der Steuereinheit und lediglich das Auswechseln der Heizauflage, welcher mit der Haut der Person in Kontakt gebracht wird, können die Betriebskosten für die Wärmeauflage gesenkt werden.

Bei einer weiteren bevorzugten Ausgestaltung umfasst die Wärmeauflage einen Vibrationserzeuger welcher ausgebildet ist, von der elektrischen Energiequelle abgegebene elektrische Energie in mechanische Energie umzuwandeln, die an den Körper der Person abgebbar ist. Der Vibrationserzeuger kann die elektrische Energie in mechanische Energie umwandeln und bspw. in Form von Vibrationen an den Körper abgeben. Der Vibrationserzeuger kann beispielsweise einen Elektromotor mir einer auf der Welle asymmetrisch aufgesetzten Masse (Unwucht) aufweisen. Wenn die Welle des Elektromotors durch die elektrische Energie angetrieben wird, beginnt die mit der Welle verbundene Masse zu rotieren. Die drehende Masse erzeugt eine Unwucht, wodurch mechanische Schwingungen bzw. mechanische Energie erzeugt wird. Die Unwucht wird durch den Versatz des Scherpunkts der Masse zur Drehachse der Welle erzeugt. Durch Kontakt der Wärmeauflage mit dem Körper der Person, kann die mechanische Energie auf die Haut bzw. den Körper der Person übertragen werden.

Die mechanische Energie ist unteranderem abhängig von der Masse bzw. der Unwucht und der Umdrehungsgeschwindigkeit des Elektromotors bzw. der Welle des Elektromotors. Je grösser die Unwucht desto mehr mechanische Energie wird erzeugt. Die erzeugte mechanische Energie ist ebenfalls umso grösser, je grösser die Umdrehungs- oder Rotationsgeschwindigkeit der Masse bzw. der Welle des Elektromotors ist.

Durch die Abgabe der mechanischen Energie an den Körper der Person, kann die Person Vibrationen wahrnehmen. Diese Vibrationen können, die Person über eine Körperfehlhaltung informieren bzw. die Körperfehlhaltung signalisieren. Durch die Vibration kann die Person aufmerksam werden und kann bspw. durch eigene bewusste Beobachtung, ihrer Körperhaltung verbessern. Fehlhaltungen können somit durch Vibrationen signalisiert werden. Die Signalisation der Fehlhaltung kann bspw. auch durch eine Wärmeauflage nahe bei der Fehlhaltung erfolgen. Ferner kann auch durch die Ausgabe von Informationen der Wärmeauflage an die Person, bspw. auf dem Display eines Mobilgerätes, die Person über Körperhaltungsfehler oder Verbesserungen der Körperhaltung informiert werden. Durch die Vibration kann die Person auch bspw. an Körperübungen (Turnübungen) zur Verbesserung der Körperhaltung erinnert werden.

Ferner schaffen Ausführungsbeispiele ein System zur Abgabe von Wärmeenergie an den Körper einer Person. Das System kann die Entspannung der Körpermuskulatur fördern. Das System umfasst mindestens eine erste Wärmeauflage gemäss beschriebenen Ausführungsbeispielen und mindestens eine zweite Wärmeauflage gemäss beschriebenen Ausführungsbeispielen und/oder ein Mobilgerät die geeignet sind, Daten mit der ersten Wärmeauflage (10) auszutauschen. In Ausführungsbeispielen kann das System ausgebildet sein, Daten von der ersten Wärmeauflage zu der zweiten Wärmeauflage zu senden und/oder zu empfangen. Die Wärmeauflagen können somit untereinander Daten austauschen und ein Netzwerk bilden. Die Wärmeauflagen können auch ausgebildet sein, Daten an ein Mobilgerät zu senden und/oder von einem Mobilgerät zu empfangen. Das Mobilgerät kann ein Mobiltelefon bzw. ein Smartphone oder ein Tablet sein oder ein speziell für die Abgabe von Wärmeenergie an den Körper einer Person ausgebildetes mobiles Gerät. Somit lässt sich ebenfalls ein Netzwerk bilden. Die Daten können Sensordaten, Körperhaltungsdaten, Steuerdaten oder weitere Daten, sein welche zum Betrieb der Wärmeauflagen wesentlich sind. Die Wärmeauflagen können auch über ein Kabel miteinander verbunden sein.

Des Weiteren wird ein Verfahren zur Steuerung einer Wärmeauflage gemäss Ausführungsbeispielen bereitgestellt, welches zur Abgabe von Wärmeenergie an den Körper einer Person vorgesehen ist. Die Wärmeauflage umfasst eine Steuereinheit und eine mit dem Körper der Person verbindbare und mit einem Heizelement versehene Heizauflage, welche elektrisch und mechanisch miteinander verbunden werden, wobei mittels eines Signalverarbeiters ein Steuersignal erzeugt wird, mittels dessen die Energieabgabe von einer Energiequelle an das Heizelement steuerbar ist.

Ferner kann mittels der Körperhaltungssensoren die Körperhaltung der Person gemessen und in dem Signalverarbeiter ermittelt und daraus das Steuersignal berechnet werden, um die Wärmeabgabe derart zu steuern, dass die Körperhaltung korrigiert wird.

Ferner wird ein Computerprogramm mit einem Programmcode zur Durchführung des oben beschriebenen Verfahrens bereitgestellt, wenn das Computerprogramm auf einem Computer oder Prozessor abläuft.

Ausführungsbeispiele der vorliegenden Erfindung werden bezugnehmend auf die beiliegenden Figuren näher erläutert. Es zeigen:

Fig. 1 eine schematische Darstellung eines ersten Ausführungsbeispiels einer Wärmeauflage in einer Draufsicht;

Fig. 2 eine schematische Darstellung eines zweiten Ausführungsbeispiels einer Wärmeauflage mit drei Verbindungselementen in einer Draufsicht;

Fig. 3a     eine weitere schematische Darstellung einer Wärmeauflage in einer Seitenansicht mit verbundener Heizauflage und Steuereinheit;

Fig. 3b     eine weitere schematische Darstellung einer Wärmeauflage in einer Seitenansicht mit getrennter Heizauflage und Steuereinheit;

Fig. 4     eine weiter schematische Darstellung einer Wärmeauflage in einer seitlichen Schnittansicht mit getrennter Heizauflage und Steuereinheit;

Fig. 5a     eine Person in einer Vorderansicht mit mehreren Wärmeauflagen;

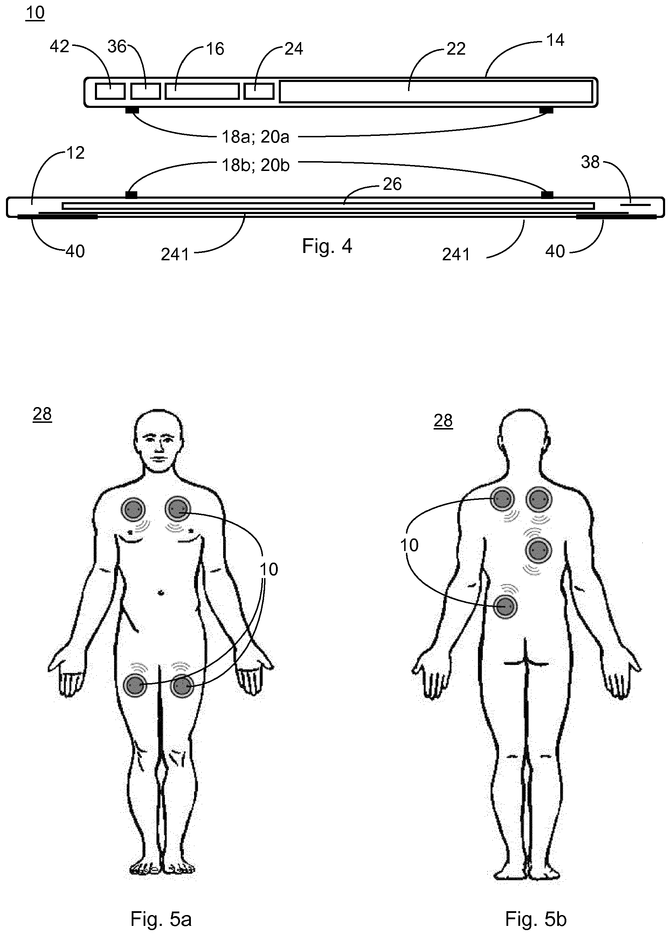

Fig. 5a     eine Person in einer Rückenansicht mit mehreren Wärmeauflagen;

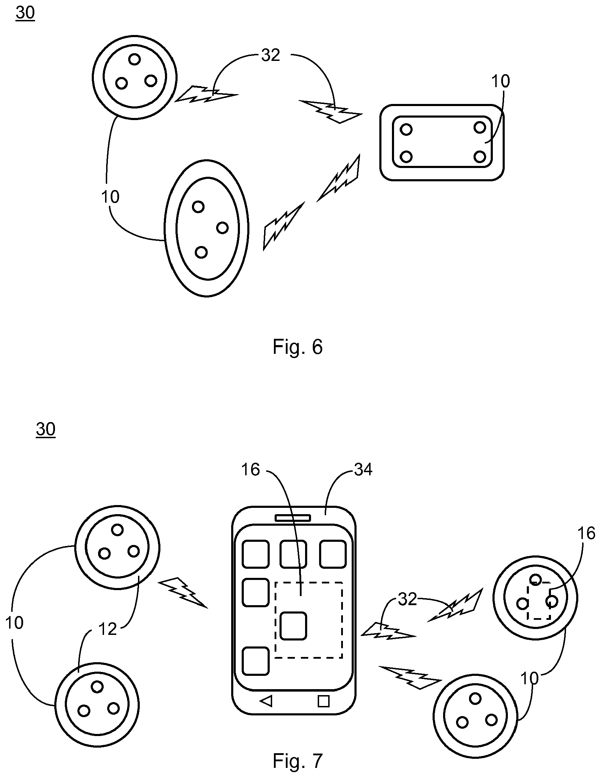

Fig. 6     ein System zur Abgabe von Wärmeenergie an den Körper einer Person mit verschiedenen Wärmeauflagen;

Fig. 7     ein System zur Abgabe von Wärmeenergie an den Körper einer Person mit einem Mobilgerät;

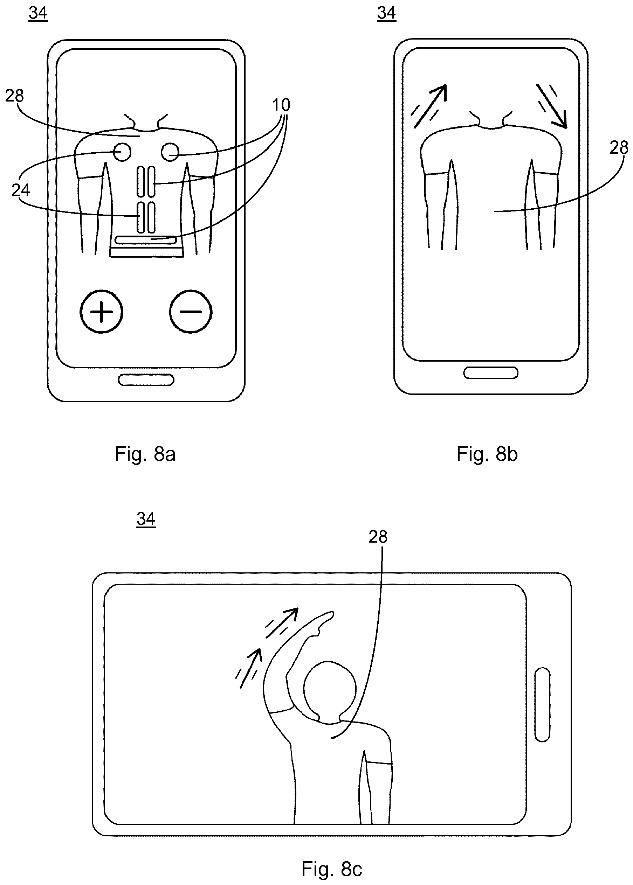

Fig. 8a-c     ein Mobilgerät mit Darstellungen zur Verbesserung der Körperhaltung;

In der nachfolgenden Beschreibung der Ausführungsbeispiele der Erfindung werden in den Figuren gleiche oder gleichwertige Elemente mit den gleichen Bezugszeichen versehen, so dass deren Beschreibung in den unterschiedlichen Ausführungsbeispiele austauschbar ist.

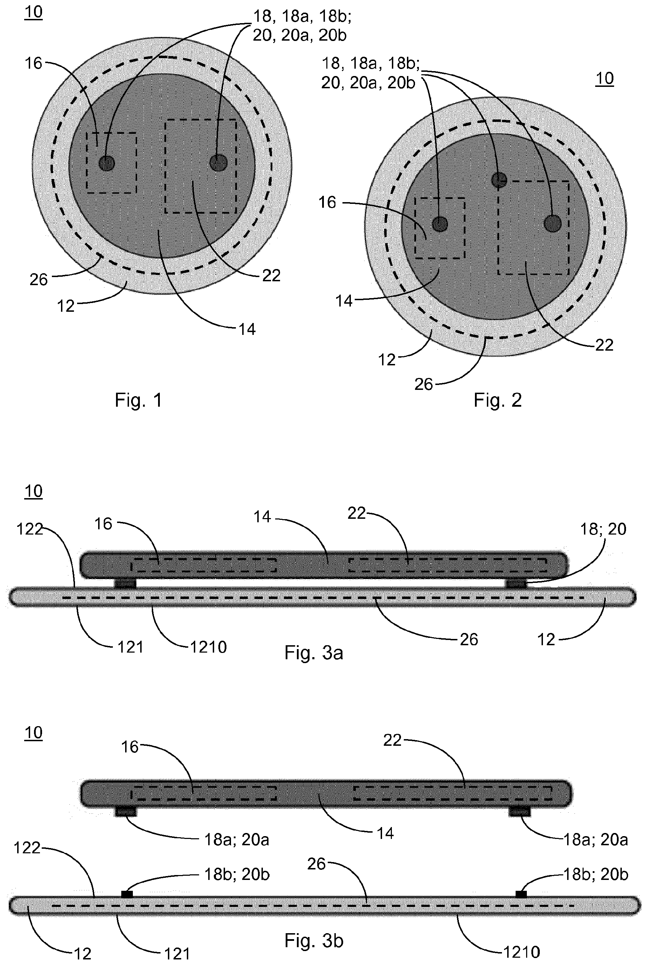

Figur 1 zeigt eine schematische Darstellung eines ersten Ausführungsbeispiels einer Wärmeauflage 10 zur Abgabe von Wärmeenergie an den Körper einer Person in einer Draufsicht. Die Wärmeauflage 10 umfasst eine mit dem Körper der Person verbindbare Heizauflage 12, die eine Oberseite und eine Unterseite aufweist und an deren Unterseite ist eine Klebefläche, zum Ankleben der Heizauflage 12 an der Hautoberfläche der Person vorgesehen. Die Klebefläche kann an einer ersten Fläche der Heizauflage ausgebildet sein. Die Klebefläche kann mit der Hautoberfläche einer Person in Kontakt gebracht werden. Beim in Kontakt bringen der Klebefläche mit der Hautoberfläche der Person sollte die Klebefläche der Heizauflage an der Haut der Person kleben bzw. haften bleiben. Die Klebefläche kann vor dem in Kontakt bringen mit der Hautoberfläche mit einer Kleberschutzschicht bspw. gegen Verschmutzung oder versehentliches Ankleben geschützt sein. Diese Schutzschicht wird vor dem Ankleben auf die Haut entfernt.

Der verwendete Klebstoff sollte hautfreundliche Eigenschaften aufweisen. Dies bedeutet, dass die Klebefläche bzw. der Klebstoff die Haut möglichst nicht schädigt oder reizt. Ferner sollte sich die Klebefläche bspw. nach dem Gebrauch der Heizauflage 12 gut von der Haut entfernen lassen. Die Klebefläche sollte mehrfach verwendbar sein.

Der Klebstoff kann vorzugsweise einen Haftklebstoff umfassen. Haftklebstoffe verfügen über keinen Verfestigungsmechanismus. Dies bedeutet, dass sie nach dem Auftragen auf ein Trägermaterial hochviskos und dauerklebrig bleiben und durch Anpressdruck auf ein Gegenstück bspw. die Haut aufgebracht werden und dort haften bleiben. Der Klebstoff an der Klebefläche der Heizauflage 12 kann gelförmig ausgebildet sein. Der Klebstoff an der Klebefläche der Heizauflage 12 kann eine hohe Viskosität aufweisen. Der Klebstoff an der Klebefläche der Heizauflage 12 kann eine gelförmige Konsistenz aufweisen. Ein gelförmiger Klebstoff kann sich leicht in Hautfalten oder Unebenheiten auf der Haut einfügen und so einen guten Klebekontakt herstellen. Der Klebstoff sollte eine gute thermische Leitfähigkeit aufweisen. Durch eine gute thermische Leitfähigkeit und einen guten thermischen Kontakt zwischen der Heizauflage 12 und der Hautoberfläche kann die Wärmeenergie gut von der Heizauflage 12 auf den Körper bzw. die Haut der Person übertragen werden.

Die Heizauflage 12 kann wenigstens ein Heizelement 26 umfassen, durch das von einer elektrischen Energiequelle 22 abgegebene elektrische Energie bzw. elektrischen Strom in Wärmeenergie bzw. thermische Energie umgewandelt wird. Das Heizelement 26 bzw. die Heizauflage 12 kann ausgebildet sein, die Wärmeenergie an den Körper der Person abzugeben bzw. zu übertragen. Das Heizelement 26 kann durch Umwandlung von elektrischer Energie in wärme Energie erwärmt werden. Die Umwandlung der elektrischen Energie bzw. des elektrischen Stroms in wärme bzw. thermische Energie kann beispielsweise durch einen elektrischen Widerstand (R) bzw. durch ein elektrisches Wärme- oder Heizelement, erfolgen. Die erzeugte thermische Energie ist mehr oder weniger proportional zur eingesetzten bzw. abgegebenen elektrischen Energie. Die elektrische Leistung (P) entspricht dabei den Gleichungen:

$$P = I^2 \times R \quad \text{oder} \quad P = \frac{U^2}{R}$$

Wobei U die elektrische Spannung und I der elektrische Strom ist. Die Leistung (P) mal die Zeit ergibt die Energie. Die Wärmeenergie kann wie z.B. bei einem Wärmepflaster, an den Körper der Person abgegeben werden. Durch die Abgabe von Wärmeenergie können sich kontraktierte Muskeln besonders gut entspannen.

Die Abgabe von Wärme kann die Blutgefässe in der Muskulatur erweitern und kann damit die Durchblutung des Muskels fördern. Durch die bessere Durchblutung können auch Schadstoffe welche den Muskel blockieren aus dem Muskel ausgeschwemmt werden und der Muskel kann sich regenerieren. Durch die Entspannung der Muskeln lösen sich Verkrampfungen und Schmerzen werden reduziert.

Das Heizelement 26 bzw. der elektrische Widerstand welches den elektrischen Strom in Wärme umwandelt, kann zentral auf der Heizauflage 12 angeordnet sein und über ein thermisch gut leitendes Material wie z.B. Metall die Wärmeenergie auf der Heizauflage 12 verteilen. Das Heizelement 26 kann aber auch flächig auf der Heizauflage 12 angeordnet sein (in der Figur 1 mit Strichlinie gezeigt). So dass die elektrische Energie auf einer grösseren Fläche auf der Heizauflage 12 in Wärmeenergie umgewandelt wird. Die Heizauflage 12 bzw. das Heizelement 26 kann eine Wärmefolie, einen Draht, ein Drahtgeflecht oder ein Draht-Gewebe umfassen. Diese können bspw. mehr oder weniger auf ihrer gesamten Fläche eine konstante Wärme abgeben.

In einer Steuereinheit 14 kann eine Energiequelle 22 fest Aufgenommen sein. Die Energiequelle 22 kann aber auch lösbar in der Steuereinheit 14 aufgenommen sein. Die Energiequelle 22 kann aber auch eine extern angeordnete Energiequelle 22 bspw. ein Stromnetz sein, welches über ein Kabel in der Steuereinheit 14 aufgenommen ist. Die Steuereinheit 14 weist ferner einen Signalverarbeiter 16 auf, der zur Abgabe eines Steuersignals vorgesehen ist, mittels dessen die Energieabgabe von der Energiequelle 22 an das Heizelement 26 steuerbar ist.

Der Signalverarbeiter 16 kann ausgebildet sein, ein Steuersignal zur Steuerung einer elektrischen Energiequelle zu erzeugen. Der Signalverarbeiter 16 kann ausgebildet sein Signale von einer oder mehrere Signalquellen bspw. Körperhaltungssensoren, Temperatursensoren oder weitere Sensoren zu empfangen und zu verarbeiten. Der Signalverarbeiter 16 kann ein Steuersignal erzeugen, mit welchem die elektrische Energiequelle gesteuert bzw. die Energiezufuhr oder Energieabgabe an den Körper bzw. die Muskulatur gesteuert wird. Der Signalverarbeiter 16 kann ein Prozessor oder eine Recheneinheit umfassen. Der Signalverarbeiter 16 kann die Signale auch teilweise oder gar nicht verarbeiten und die Signale zur Verarbeitung an einen weiteren Signalverarbeiter bspw. in einem Mobilgerät oder an einer weiteren Wärmeauflage 10 bzw. Steuereinheit 14 weiterleiten. Dabei kann der Signalverarbeiter 16 bspw. lediglich Funksignale empfangen und/oder aussenden.

Das Heizelement 26 kann ausgebildet sein, den elektrischen Strom von der elektrischen Energiequelle 22 direkt zu empfangen und die Wärmeenergie an die Haut der Person abzugeben. Das Heizelement 26 kann auch ausgebildet sein, den elektrischen Strom von der elektrischen Energiequelle 22 durch den Signalverarbeiter 16 zu empfangen und die Wärmeenergie an die Haut der Person abzugeben. Ferner kann der Signalverarbeiter 16 das Steuersignal zur Steuerung der elektrischen Energiequelle 22 an die Heizauflage 12 übertragen und die Heizauflage 12 kann das Signal an die elektrische Energiequelle 22 übertragen und die elektrische Energie von der elektrischen Energiequelle 22 empfangen.

Die elektrische Energiequelle 22 und das Heizelement 26 können ausgebildet sein die Heizauflage 12 in wenigen als 1 min vorzugsweise in weniger als 20 sek., besonders bevorzugt in weniger als 5 sek. um 20° C zu erwärmen. Die Erwärmung der Heizauflage 12 kann durch einen Temperatursensor überwacht werden, um Hautverletzungen, wie bspw. Verbrennungen zu vermeiden. Der Temperatursensor kann an der Heizauflage 12 angeordnet sein und bspw. die Temperatur der Heizauflage 12 überwachen. Der Signalverarbeiter 16 kann bspw. einen Messwert des Temperatursensors verarbeiten und abhängig von dem am Temperatursensor gemessen Messwert oder Temperaturwert das Steuersignal zur Steuerung der elektrischen Energiequelle 22 verändern, sodass mehr elektrische Energie der Heizauflage 12 zugeführt wird, wenn die Heizauflage 12 sich stärker erwärmen soll und weniger oder keine elektrische Energie der Heizauflage 12 zugeführt wird, wenn sich die Heizauflage 12 weniger oder gar nicht erwärmen soll. Der Temperatursensor kann auch die Hauttemperatur bzw. Körpertemperatur der Person messen und abhängig von der Körpertemperatur die elektrische Energie wie vorhergehend beschrieben steuern, so dass die Wärmeauflage 10 die Person wärmt, sobald ein vorgegebener Temperaturwert der Hauttemperatur bzw. Körpertemperatur unterschritten wird.

Die elektrische Energiequelle 22 kann z.B. eine mobile elektrische Energiequelle wie bspw. eine Batterie oder einem Akku sein. Die Batterie bzw. der Akku kann elektrische Energie bzw. elektrischen Strom abgegeben. Ein Akku kann über eine weitere Energiequelle und eine Ladeschaltung mit elektrischer Energie aufgeladen oder nachgeladen werden. Die elektrische Energiequelle 22 kann auch ein öffentliches Stromnetz sein, wobei z.B. über ein Netzteil die Spannung des Stromnetzes vor der Einspeisung in die Wärmeauflage 10 reduziert werden kann.

Sowohl die Heizauflage 12 als auch die Steuereinheit 14 sind in der Figur 1 rund dargestellt. Die Heizauflage 12 und die Steuereinheit 14 könne aber auch eine ovale oder n-Eckige Form aufweisen. Sowohl die Heizauflage 12 als auch die Steuereinheit 14 können auch eine viereckige oder quadratische Form bspw. mit spitzen oder runden Ecken aufweisen. Sowohl die Heizauflage 12 als auch die Steuereinheit 14 können eine beliebige Form aufweisen. Die Heizauflage 12 und die Steuereinheit 14 können auch unterschiedliche Formen aufweisen. Bspw. kann die Steuereinheit 14 rund sein und die Heizauflage 12 weist eine längliche viereckige Form auf.

Die die Heizauflage 12 und die Steuereinheit 14 sind durch wenigstens einen elektrischen Verbinder

18, über den die elektrische Energie zum Heizelement 26 übertragbar ist, und wenigstens einen mechanischen Verbinder 20, elektrisch und mechanisch miteinander verbindbar.

Auf der Steuereinheit 14 kann ein erstes elektrisches Verbindungselement 18a zur Übertragung der elektrischen Energie bzw. eines elektrischen Strom von der Steuereinheit 14 auf ein dazu komplementäre zweites elektrisches Verbindungselement 18b, welches an der Heizauflage 12 ausgebildet ist, angeordnet sein. Die elektrischen Verbindungselemente 18a, 18b bzw. der elektrische Verbinder 18 kann mit der elektrischen Energiequelle, dem Signalverarbeiter 16 oder bspw. einem Steuerelement, welches das Steuersignal zur Steuerung der elektrischen Energiequelle 22 empfängt, verbunden sein. Der elektrische Verbinder 18 kann auch ein Steuersignal oder Sensorsignal übertragen. Der elektrische Verbinder 18 kann bspw. als elektrischer Stecker und elektrische Buchse oder Magnete ausgebildet sein. Die elektrischen Verbindungselemente 18a, 18b sind vorzugsweise aus Metall. Die elektrischen Verbindungselemente 18a, 18b können zusätzlich eine Beschichtung mit einem anderen Metall aufweisen, welches einen kleinen Übergangswiderstand aufweist bzw. nicht oxidiert (bspw. Gold). Die elektrischen Verbindungselemente 18a, 18b zur Übertragung der elektrischen Energie können mechanisch fix an der Steuereinheit 14 und/oder an der Heizauflage 12 angeordnet sein. Die elektrischen Verbindungselemente 18a, 18b können aber auch über ein flexibles Kabel mit der Steuereinheit 14 und/oder der Heizauflage 12 verbunden sein.

Auf der Steuereinheit 14 kann mindestens ein erstes mechanisches Verbindungselement 20a angeordnet sein. Zu dem ersten mechanische Verbindungselement 20a, welches an der Steuereinheit 14 angeordnet ist, kann an der Heizauflage 12 ein komplementäres zweites mechanisches Verbindungselement 20b angeordnet sein. Das zweite mechanische Verbindungselement 20b kann an der Heizauflage 12 an der, der Klebefläche gegenüberliegenden Oberseite an der Heizauflage 12 angeordnet sein. Das erste mechanische Verbindungselement 20a an der Steuereinheit 14, sollte auf die Position das zweite mechanische Verbindungselement 20b an der Heizauflage 12 ausgerichtet sein, so dass sich die mechanischen Verbindungselemente 20a, 20b leicht verbinden lassen.

Durch einen mechanischen Verbinder 20 bzw. die mechanischen Verbindungelemente 20a, 20b, lassen sich die Steuereinheit 14 und die Heizauflage 12 einfach zu einer Wärmeauflage 10 zusammenführen. Umgekehrt lässt sich die Wärmeauflage 10 durch den mechanischen Verbinder 20 in eine Steuereinheit 14 zur Verwendung in einer Wärmeauflage 10 gemäss Ausführungsbeispiel und in eine Heizauflage 12 zur Verwendung in einer Wärmeauflage 10 gemäss Ausführungsbeispiel aufteilen bzw. trennen. In der Figur 1 werden die Steuereinheit 14 und die Heizauflage 12 durch zwei mechanische Verbinder 20 verbunden.

Die Heizauflage 12 kann ein Wegwerfelement sein, welches sich nach einmaligem oder mehrmaligem ca. zehnmaligen oder ca. zwanzigmaligen oder ca. dreissigmaligen Gebrauch ersetzen lässt. Dies hat zum einen Vorteile bezüglich der Hygiene, da die Heizauflage 12 direkt auf den Körper bzw. die Haut der Person aufgeklebt wird und teils mit viel Körperschweiss und Fett in Berührung kommt. Zum anderen kann beim Nachlassen der Klebewirkung die Klebefläche der Heizauflage 12 durch Auswechseln der Heizauflage 12 wieder eine gute Klebewirkung der Wärmeauflage 10 am Körper der Person erzielt werden. Dabei ist es auch möglich, dass z.B. das Heizelement 26, welches die elektrische Energie in wärme Energie umwandelt, aus einer verbrauchten Heizauflage 12 entfernt und in eine andere, neue Heizauflage 12 eingesetzt wird. Durch wiederverwenden des Heizelementes 26 oder von Sensoren an der Heizauflage 12 können Kosten und Ressourcen eingespart werden.

Die Steuereinheit 14 kann ein wiederverwendbares Element sein. Die Steuereinheit 14 kann z.B. den Signalverarbeiter 16, eine Funkeinheit, die elektrische Energiequelle 22 oder weiter Elektronik umfassen. Die Steuereinheit 14 sollte Bauteile umfassen, welche sich gut wiederverwerten lassen und/oder teuer bzw. wertvoll sind. Die gesamte Intelligenz bzw. die zur Intelligenz der Wärmeauflage 10 beitragenden Bauteile können auf der Steuereinheit 14 angeordnet sein. Die Steuereinheit 14 kann beliebig oft wiederverwendet werden, wodurch Kosten- und Ressourcen eingespart werden können.

Die Steuereinheit 14 kann derart an der Heizauflage 12 angeordnet sein, dass die Steuereinheit 14 beim Tragen bzw. im an den Körper einer Person angeklebten Zustand der Wärmeauflage 10 nicht direkt mit der Haut der Person in Berührung kommt. Dadurch entstehen weniger unhygienische Verunreinigungen an der Steuereinheit 10 bspw. durch Schweiss oder Körperfett.

Der elektrische Verbinder 18 weist ein mit der Steuereinheit 14 verbundenes erstes elektrisches Verbindungselement 18a und ein mit der Heizauflage 12 verbundenes zweites Verbindungselement 18b auf. Der mechanische Verbinder 20 weist ein mit der Steuereinheit 14 verbundenes erstes mechanisches Verbindungselement 20a und ein mit der Heizauflage 12 verbundenes zweites mechanisches Verbindungselement 20b auf. Das erste und zweite mechanische Verbindungselement 20a, 20b kann das dazu korrespondierende erste bzw. zweite elektrische Verbindungselement 18a, 18b umschliessen. Das mechanische Verbindungselement 20a, 20b kann das elektrische Verbindungselement 18a, 18b zur Übertragung des elektrischen Stroms oder eines Steuersignal oder eines Sensorsignales umfassen. Mit anderen Worten kann das elektrische Verbindungselement 18a, 18b zur Übertagung des elektrischen Stroms z.B. in das mechanische Verbindungselement 20a, 20b integriert sein. Das mechanische Verbindungselement 20a, 20b kann auch z.B. in das elektrische Verbindungselement 18a, 18b zur Übertagung des elektrischen Stroms integriert sein. Bspw. ein metallischer Druckknopf mit entsprechendem Gegenstück oder ein metallischer Magnet, kann sowohl als mechanischer Verbinder 20 bzw. mechanisches Verbindungselement 20a, 20b wie auch als elektrischer Verbinder 18 bzw. elektrisches Verbindungselement 18a, 18b zur Übertragung des elektrischen Stroms genutzt werden.

Mit anderen Worten: Die Wärmeauflage 10 bzw. das smarte Wärmepflaster kann selbstklebend sein und kann als Heizelement in der Heizauflage 12 eine Wärmefolie oder Drahtgeflecht besitzen, welche durch Energie innert Sekunden auf die therapeutische Wärme von 40° C erhitzt werden kann. Die selbstklebende Folie kann zwanzig- bis dreissigmal verwendet werden.

Figur 2 zeigt eine Wärmeauflage 10 mit drei mechanischen Verbinder 20 bzw. mechanischen Verbindungselementen 20a, 20b. Die Wärmeauflage 10 kann mindestens drei mechanische Verbindungselemente 20a, 20b umfassen, wobei an der Steuereinheit 14 drei erste mechanische Verbindungselemente 20a und an gleicher Stelle gegenüberliegend an der Heizauflage 12 drei komplementäre zweite mechanische Verbindungselemente 20b zum Verbinden der Heizauflage 12 mit der Steuereinheit 14 ausgebildet sind. Die mechanischen Verbinder 20 können auch die elektrischen Verbinder 18 bzw. die elektrischen Verbindungselemente 18a, 18b umfassen. Die mechanischen Verbindungselemente 20a, 20b können somit spiegelverkehrt zwischen dem Steuereinheit 14 und der Heizauflage 12 angeordnet sein.

Durch die Positionierung der mechanischen Verbinder 20 kann eine Ausrichtungscodierung erreicht werden, sodass sich bspw. die Steuereinheit 14 nur in einer bestimmten Position oder Lage mit der Heizauflage 12 verbinden lässt (Codierung). Dadurch können bspw. bestimmte Kräfte, bspw. vertikal Kräfte, welche beim Tragen am Körper einer Person auf die Wärmeauflage 10 wirken, besser aufgenommen werden. Ferner kann durch eine Codierung bei Verbindungselementen 18a, 18b; 20a, 20b, welche sowohl mechanische 20a, 20b wie auch elektrische Verbindungselemente 18a, 18b umfassen die Gefahr einer elektrischen Falsch-Polung beim Verbinden der Heizauflage 12 mit der Steuereinheit 14 reduziert werden. Ferner kann bei drei Verbinder 18; 20 nebst zwei elektrische Polen auf dem dritten Verbindungselement ein Steuersignal oder Sensorsignal übertragen werden. Es können aber auch mehr als drei bspw. vier oder mehr mechanische Verbindungselemente 20a, 20b und/oder elektrische Verbindungselemente 18a, 18b, die Heizauflage 12 und die Steuereinheit 14 zu einer Wärmeauflage 10 verbinden.

Figur 3a und 3b zeigt eine weitere Darstellung eines Ausführungsbeispiels der Wärmeauflage 10 in einer Seitenansicht. Figur 3a zeigt die Wärmeauflage 10 mit verbundener bzw. gekoppelter Heizauflage 12 und Steuereinheit 14. Im gekoppelten Zustand kann in der Darstellung nicht zwischen elektrischem oder mechanischen Verbinder 18; 20 bzw. elektrischen oder mechanischen Verbindungselementen 18a, 18b; 20a, 20b, welche an der Steuereinheit 14 oder an der Heizauflage 12 befestigt sind, unterschieden werden.

Die mit dem Körper der Person verbindbare Heizauflage 12 weist eine Oberseite 122 und eine Unterseite 121 auf und auf deren Unterseite 121 ist die Klebefläche 1210, zum Ankleben der Heizauflage 12 an der Hautoberfläche der Person vorgesehen. Die Heizauflage 12 umfasst wenigstens das Heizelement 26. Durch das Heizelement 26 kann von der elektrischen Energiequelle 22 abgegebene elektrische Energie in Wärmeenergie umgewandelt werden. Ferner umfasst die Wärmeauflage die Steuereinheit 14, welche zur festen oder lösbaren Aufnahme der Energiequelle 22 vorgesehen ist und die einen Signalverarbeiter 16 aufweisen kann der zur Abgabe eines Steuersignals vorgesehen ist, mittels dessen die Energieabgabe von der Energiequelle 22 an das Heizelement 26 steuerbar ist. Die Heizauflage 12 und die Steuereinheit 14 sind durch wenigstens einen elektrischen Verbinder 18, über den die elektrische Energie zum Heizelement 26 übertragbar ist, und wenigstens einen mechanischen Verbinder 20, elektrisch und mechanisch miteinander verbindbar.

Figur 3b zeigt die Wärmeauflage 10 mit getrennter Heizauflage 12 und Steuereinheit 14. An der Steuereinheit 14 sind dabei je zwei erste elektrische Verbindungselemente 18a und zwei erste mechanische Verbindungselemente 20a gezeigt. Das erste mechanische Verbindungselement 20a umschliesst bzw. umfasst dabei das erste elektrische Verbindungselement 18a zur Übertragung des elektrischen Stroms oder eines Steuersignals. Die Verbindungselemente können somit erste mechanische 20a und/oder elektrische 18a Verbindungselemente sein. An der Heizauflage 12 können zu den ersten Verbindungselementen 18a, 20a der Steuereinheit 14 komplementäre zweite elektrische Verbindungselemente 18b und zweite mechanische Verbindungselemente 20b angeordnet sein. Das zweite mechanische Verbindungselement 20b umschliesst bzw. umfasst in der Darstellung das zweite elektrische Verbindungselement 18b zur Übertragung des elektrischen Stroms oder eines Steuersignals.

Die Klebefläche 1210 kann an einer Unterseite 121 bzw. ersten Fläche 121 der Heizauflage 12 ausgebildet sein. An einer gegenüberliegenden Oberseite der Heizauflage 122 bzw. zweiten Fläche können die elektrischen oder mechanischen Verbindungselemente 18b, 20b angeordnet sein. Die Heizauflage 12 umfasst wiederum wenigstens das Heizelement 26. Durch das Heizelement 26 kann von der elektrischen Energiequelle 22 abgegebene elektrische Energie in Wärmeenergie umgewandelt werden. Die Steuereinheit 14 kann den Signalverarbeiter 16 zum Steuern der Energieabgabe von der Energiequelle 22 an das Heizelement 26 aufweisen.

Figur 4 zeigt eine weiter schematische Darstellung einer Wärmeauflage in einer seitlichen Schnittansicht mit getrennter Heizauflage 12 und Steuereinheit 14. In der Schnittansicht sind diverse Elemente gezeigt, welche die Steuereinheit 14 und die Heizauflage 12 umfassen können.

In oder an der Steuereinheit 14 kann bspw. die elektrische Energiequelle 22 angeordnet oder befestigt sein. Die elektrische Energiequelle 22 kann auch anordenbar oder befestigbar sein. Dies bedeutet, die elektrische Energiequelle 22 kann fest mit der Steuereinheit 14 verbunden oder entfernbar sein. Die elektrische Energiequelle 22 kann ausgebildet sein, die Heizauflage 12 und/oder die Steuereinheit 14 mit elektrischer Energie zu versorgen. Dadurch ist die Wärmeauflage 10 unabhängig von einer leitungsgebundenen elektrischen Stromversorgung, welche z.B. mit einer Steckdose verbunden werden müsste und die Bewegungsfreiheit der Person einschränkt. Die mobile elektrische Energiequelle 22 kann z.B. eine Batterie oder einem Akku sein. Die Batterie bzw. der Akku kann elektrische Energie bzw. elektrischen Strom abgegeben. Ein Akku kann über eine weitere Energiequelle und eine Ladeschaltung mit elektrischer Energie aufgeladen oder nachgeladen werden. Die Wärmeauflage 10 kann in Ausführungsbeispielen zur Energieversorgung auch bspw. über ein Netzteil mit einer Steckdose verbunden werden. Die Wärmeauflage 10 kann somit mit einer extern angeordneten Energiequelle verbindbar sein. Durch die Versorgung der Wärmeauflage 10 mit elektrischer Energie aus einer Steckdose muss die elektrische Energiequelle nicht ausgewechselt oder Nachgeladen werden.

Die Wärmeauflage 10, vorzugsweise die Steuereinheit 14 kann einen Körperhaltungssensor 24 umfassen, wobei der Körperhaltungssensor 24 ausgebildet sein kann die Körperhaltung und/oder Bewegung der Person zumindest in Teilen zu erfassen und Körperhaltungsdaten zu erzeugen. Der Signalverarbeiter 16 kann ausgebildet sein, mittels der Körperhaltungsdaten die Energieabgabe von der Energiequelle 22 an das Heizelement 26 zu steuern. Die Wärmeauflage 10 kann lediglich ein Körperhaltungssensor 24 oder mehrere Körperhaltungssensoren 24 umfassen. Dabei können mehreren Körperhaltungssensoren 24 die Körperhaltung in der Regel präziser erfassen. Der Körperhaltungssensor 24 oder der Signalverarbeiter 16 kann erkennen, ob bei der Person eine schlechte oder falsche Körperfehlhaltung vorliegt oder ob die Körperhaltung korrekt oder eher korrekt ist. Die Körperhaltungsdaten können aufgrund der Körperhaltung erzeugt werden. Die Körperhaltungsdaten können ein analoges oder digitales elektrisches Signal sein, welches vom Körperhaltungssensor 24 erzeugt wird. Das Signal kann als leitungsgebundenes Signal auf einer elektrischen Leitung oder leitungsungebundenes Signal (Wireless) bzw. Funksignal vom Körperhaltungssensor 24 bereitgestellt bzw. ausgegeben werden.

Der Signalverarbeiter 16 kann ausgebildet sein, eine ermittelten Körperhaltung mit einer Sollkörperhaltung und/oder einen ermittelten Bewegungsablauf mit einem Sollbewegungsablauf zu vergleichen und abhängig von der Abweichung zwischen den ermittelten Körperhaltung und der Sollkörperhaltung und/oder dem ermittelten Bewegungsablauf und dem Sollbewegungsablauf das Steuersignal zu erzeugen. Die Sollkörperhaltung oder der Sollbewegungsablauf kann Personen spezifisch abgespeichert sein. Der Vergleich zwischen einem Soll- und einer ermittelten Wert ergibt eine Differenz, wobei durch die Abgabe von Energie an den Körper und die daraus resultierende Entspannung der Muskulatur die Differenz zwischen Sollwert und einer ermittelten Wert reduziert werden kann.

Der Körperhaltungssensor 24 kann am oder in der Steuereinheit 14 angeordnet sein. Der Körperhaltungssensor 24 kann die Körperhaltung auch mit Hilfe von Hautdaten identifizieren bzw. erfassen. Der Körperhaltungssensor 24 kann einen 3D-Gyro-Sensor (Gyroskop 3D), Beschleunigungssensor, Temperatursensor, Biegesensor, EMG-Sensor, Dehnmessstreifen oder weitere Sensoren zur Erfassung der Körperhaltung umfassen. Durch den Körperhaltungssensor 24 kann die Körperhaltung der Person erfasst werden. Körperhaltungssensoren 24 können z.B. an den Schultern oder am Nacken oder im Bereich der Wirbelsäule angeordnet sein und die Körperhaltung der Person erfassen. Ein 3D-Gyro-Sensor bzw. ein 3D-Gyroskop wie bspw. der ITG-3200 von IvenSens Inc. kann Bewegungen in 3-Achsen erfassen und somit die Position im Raum bestimmen. Durch einen Beschleunigungssensoren bzw. einen Accelerometer-Sensorwie bspw. BMA400 von Bosch kann eine Veränderung der Position im Raum erfasst werden. Es gibt auch Sensoren, welche die beide vorhergehend beschriebene Funktionen vereinen, bspw. den MO 416.H1X oder den MO416.H13 von Makersan.

Vorzugsweise an der Heizauflage 12, welche näher am Körper angeordnet ist, als die Steuereinheit 14, können als Körperhaltungssensor 24 auch ein Biegesensor 241 angeordnet werden. Biegesensoren 241 können die Körperhaltung insbesondere im Bereich der Schulter und der Wirbelsäule erfassen und eine Krümmung der Wirbelsäule oder ein hochziehen oder abfallen der Schultern gut erfassen. Der Biegesensor 241 kann eine Länge von 5 cm bis 40 cm aufweisen. Vorzugsweise weisen Biegesensoren 241 eine Länge von 10 cm bis 30 cm auf. Lange Biegesensoren 241 können bspw. im Bereich der Wirbelsäule angeordnet werden und dies über deren gesamte Länge vom Steissbein bis zu den Halswirbeln erfassen. Ferner können Biegesensoren 241 die Körperhaltung an den Schultern bspw. vom linken Arm bis zum rechten Arm erfassen. Auch kann je ein Biegesensor 241 an der linken Schulter und an der rechten Schulter angeordnet sein. Es können anstelle von einzelnen langen Biegesensoren 241 auch mehrere kürzere Biegesensoren 241 mit einer Länge von bspw. 1 cm bis 5 cm vorzugsweise 2 cm bis 3 cm bspw. an den beschriebenen Stellen angeordnet werden. Mit einem Biegesensor 241 wie bspw. dem SEN-10264 von Spectra Symbol können Krümmungen bspw. der Wirbelsäule oder ein hochziehen oder abfallen der Schultern erfasst werden. Bei dem Biegesensor handelt sich um eine schmale Leiste mit 7,3 cm Länge und 6,3 mm Breite. Im Ruhezustand beträgt der angelegte Widerstand 25 kOhm. Je stärker der Sensor gebogen wird, desto mehr steigt der Widerstand, der maximal 125 kOhm betragen kann.

Somit können mit Körperhaltungssensoren 24 bspw. Fehlhaltungen in der Körperhaltung erfasst werden. Fehlhaltungen in Bezug auf die Körperhaltung sind bspw. ein Buckel, ein Hohlkreuz, Schulterspannung, Schieflage der Schultern oder des Rückens. Aus den Messwerten der verschieden Körperhaltungssensoren lässt sich dann die Körperhaltung der Person bestimmen.

Ferner kann die Steuereinheit 14 den Signalverarbeiter 16 umfassen. Der Signalverarbeiter 16 kann am oder in der Steuereinheit 14 angeordnet sein. Der Signalverarbeiter 16 kann bspw. ein Prozessor oder eine Prozessoreinheit sein. Der Prozessor kann auch bspw. in einem auf die Verbesserung der Körperhaltung spezialisierten Gerät, insbesondere auf einem mobilen Gerät integriert sein. Der Prozessor kann auch in einem universellen Mobilgerät wie z.B. einem Tablet oder einem Smartphone oder einem anderen mobilen Endgerät integriert sein. Spezialisierte oder universelle Mobilgeräte lassen sich oftmals nicht mehr unterscheiden, da Funktionen häufig über die Software im Mobilgerät bereitgestellt werden können. Spezialisierte oder universelle Mobilgeräte umfassen üblicherweise mindestens eine Eingabeeinheit, ein Display, einen Prozessor, Sensoren, Kommunikationsmittel sowie einen Energiespeicher.

Abhängig von den Körperhaltungsdaten bzw. dem Signal des Körperhaltungssensors 24 kann der Signalverarbeiter 16 die Körperhaltung bestimmen. Abhängig von der durch den Signalverarbeiter 16 bestimmten Körperhaltung, kann der Signalverarbeiter 16 ein Steuersignal zur Steuerung einer elektrischen Energiequelle 22 erzeugen. Das Steuersignal zur Steuerung der elektrischen Energiequelle 22 kann wiederum ein analoges oder digitales elektrisches Signal sein, welches vom Signalverarbeiter 16 erzeugt wird. Das Signal kann als leitungsgebundenes Signal auf einer elektrischen Leitung oder leitungsungebundenes Signal (Wireless) bzw. Funksignal vom Signalverarbeiter 16 bereitgestellt bzw. ausgegeben werden. Der Signalverarbeiter 16 kann auch direkt mehr oder weniger elektrische Energie ausgeben, welche von einer elektrischen Energiequelle 22 erzeugt bzw. gespeichert wurde.

Der Signalverarbeiter 16 kann ausgebildet sein, die Körperhaltungsdaten zu empfangen. Der Signalverarbeiter 16 kann ausgebildet sein die Körperhaltungsdaten als Signal bzw. elektrisches Signal zu empfangen. Das Signal kann ein leitungsgebundenes Signal auf einer elektrischen Leitung oder leitungsungebundenes Signal (Wireless) bzw. Funksignal sein. Vorzugseise ist der Empfang des Signals beim Signalverarbeiter 16 auf die Ausgabe des Signals des oder der Körperhaltungssensoren 24, 241 abgestimmt.

Die Wärmeauflage 10 bzw. die Steuereinheit 14 kann eine Funkeinheit 36 zum Senden und/oder Empfangen von Daten umfassen. Die Funkeinheit kann auf der Steuereinheit 14 angeordnet sein. Die Funkeinheit 36 kann auch bspw. in dem Signalverarbeiter 16 oder einem Prozessor integriert bzw. angeordnet sein. Die Funkeinheit 36 kann Daten wie Bspw. Körperhaltungsdaten, Sensordaten, Betriebsdaten, Steuerdate usw. zwischen Wärmeauflagen 10 und/oder einem Mobilgerät austauschen. Die gesamte Elektronik (ausser einiger Sensoren) kann sich auf der Steuereinheit 14 befinden. An der Steuereinheit können erste elektrische und mechanische Verbindungselement 18a; 20a angeordnet sein zum Verbinden der Steuereinheit 14 mit der Heizauflage 12.

Die Heizauflage 12 kann ausgebildet sein elektrischen Strom von der elektrischen Energiequelle 22 oder von dem Signalverarbeiter 16 zu empfangen. Die Heizauflage 12 kann über elektrische Leitung welche die elektrische Energie bzw. den Strom übertragen können mit der elektrischen Energiequelle 22 bzw. dem Signalverarbeiter 16 verbunden sein. Die Heizauflage 12 kann Temperatursensor 38, Biegesensor 241, Elektroden 40, Heizelement 26 (Heizfolie/Heizdrahtgeflecht), Klebefläche 1210 mit Klebstoff und zweite elektrische und mechanische Verbindungselement 18b; 20b umfassen.

Die Heizauflage 12 kann die elektrische Energie zumindest teilweise in thermische Energie umwandeln und an den Körper der Peron abgeben. Die abgegebene thermische Energie kann entspannend bzw. stimulierend auf die Muskulatur wirken und kann die Körperhaltung der Person verbessern.

Die Energie welche durch eine oder mehrere Heizauflage 12 an den Körper abgegeben wird, kann auch weiteres Körpergewebe wie z.B. Fettgewebe beeinflussen. Insbesondere durch die Kontraktion der Muskeln kann die Verbrennung von Fettgewebe im Körper aktiviert werden. Auch dadurch kann das Wohlbefinden der Person gesteigert und die Körperhaltung verbessert werden.

Das Heizelement 26 oder das Wärmeelement in der Heizauflage 12 kann bspw. eine Folie umfassen, wobei die Folie mehr oder weniger auf ihrer ganzen Fläche elektrische Energie in thermische Energie umwandeln kann. Als Wärmefolie kann z.B elektrisch beheizbare Polyamid-Folie wie bspw. die RS Folie von Kapton verwendet werden. Die Wärmefolie ermöglichen einen konstanten und effektiven Wärmeaustausch. Das Heizelement 26, kann auch z.B. elektrische Leiter bzw. ein Drahtgeflecht umfassen und ausgebildet sein, konstante Wärme, bei einem niedrigeren Energiebedarf abzuliefern. Das Heizelement 26 kann auch auf relativ kleinem Raum die elektrische Energie in wärme Energie umwandeln und über einen guten thermischen Leiter, wie z.B. ein Metallplättchen, die Wärme auf einer grösseren Fläche im oder an der Heizauflage 12 verteilen.

An der Heizauflage 12 können Elektroden 40 angeordnet sein und der Signalverarbeiter 16 kann ausgebildet sein durch Elektromyografie die Muskelspannung zu bestimmen. Durch die Elektroden 40 lassen sich Potentialänderungen eines Muskels oder einer Muskelgruppe messen. Der Signalverarbeiter 16 kann ein Steuersignal zur Steuerung des elektrischen Energiespeicher 22 erzeugen welches abhängig ist von der Muskelspannung. Bei Verspannung des Muskels kann z.B. die Wärmeauflage 10 durch Abgabe elektrischer Energie, von dem elektrischen Energiespeicher 22 an die Heizauflage 12, erwärmt werden. Durch die Erwärmung können Muskeln stimuliert werden.

Mit den Elektroden 40 kann auch der Hautwiderstand bestimmt werden. Dabei kann bspw. durch einen Strom welcher von einer ersten Elektrode 40 zu einer zweiten Elektrode 40 fliesst, durch messen der Spannung zwischen der beiden Elektroden der Leitwert, der Widerstand bzw. die Impedanz zwischen den Elektroden 40 und damit der Haut der Person bestimmte werden. Der Leitwert oder der Widerstandswert kann ebenfalls zur Steuerung der Wärmeenergie durch den Signalverarbeiter 16, an der Wärmeauflage 10, verwendet werden.

Die Wärmeauflage 10 kann eine Vibrationserzeuger 42 umfassen, welcher ausgebildet ist, von der elektrischen Energiequelle 22 abgegebene elektrische Energie in mechanische Energie umzuwandeln und mechanische Energie an den Körper der Person abzugeben. Mechanische Energie kann in Form von Vibrationen an die Haut bzw. den Körper der Person abgegeben werden. Die Vibrationen können durch einen Vibrationserzeuger 42 welcher z.B. einen Elektromotor und ein Unwucht umfasst erzeugt werden. Bei einer Unwucht liegt der Schwerpunkt nicht auf der Rotationsache. Der Vibrationserzeuger kann auch ein Linearmotor sein, der eine Masse hin und her bewegt, und dadurch Vibrationen erzeugt. Die Vibrationen können der Person einen Hinweis bspw. zur Körperhaltung geben oder auch Muskeln durch Massage entspannen bzw. lockern.

Die Wärmeauflage 10 bzw. die Heizauflage 12 kann auch einen Temperatursensor 38 umfassen. Der Temperatursensor 38 kann abhängig von der Anordnung an der Wärmeauflage 10 oder an der Heizauflage 12 die Hauttemperatur bzw. Körpertemperatur der Person erfassen. Der Temperatursensor 38 kann auch die Temperatur der Heizauflege 12 erfassen. Der Temperatursensor 38 kann mit dem Signalverarbeiter 16 verbunden sein und aufgrund des gemessene Temperaturwerts des Temperatursensors 38 dem Signalverarbeiter 16 Temperaturdaten zur Verfügung stellen. Der Signalverarbeiter 16 kann abhängig von den Temperaturdaten das Steuersignal zur Steuerung der elektrischen Energiequelle 22 erzeugen. Somit kann abhängig von dem durch den Temperatursensor 38 erfassten Temperaturwert die Energieabgabe von der Energiequelle 22 an das Heizelement 26 steuerbar sein bzw. die elektrische Energie zufuhr für die Heizauflege 12 gesteuert werden.

Figur 5a und 5b zeigen eine Person 28 mit mehreren am Körper angeklebten bzw. befestigten Wärmeauflagen 10. Figur 5a zeigt die Person in einer Voransicht. Je eine Wärmeauflage 10 ist am rechten und linken Oberschenkel angeordnet. Zwei weitere Wärmeauflagen 10 sind oberhalb der Brust angeordnet. Die Wärmeauflagen 10 können an beliebigen Stellen am Körper angeordnet bzw. aufgeklebt werden. Vorzugsweise können die Wärmeauflagen 10 auf der Haut über darunterliegenden Muskel oder Muskelgruppen welche verkrampft oder verspannt sind angeordnet werden. Die Wärme der Wärmeauflagen 10 kann helfen die Muskeln zu entspannen. Die Wärmeauflage 10 kann bspw. auch an allen Trigger-Points des Körpers verwendet bzw. angeordnet werden (bspw. Oberschenkel, Rücken, etc.)

Der Wärme werden in der Medizin vor allem folgende Wirkungen zugesprochen: Muskelentspannung, Verbesserung der Durchblutung, Verminderung der Viskosität der Gelenkflüssigkeit, Verbesserung der Dehnbarkeit des kollagenen Bindegewebes und Schmerzlinderung. Die Wärmeauflagen 10 können den Schmerzen bzw. die Verspannungen dort bekämpfen bzw. lindern, wo sie stattfinden. Wärmetherapien sollten nicht durchgeführt werden bei entzündlichen Prozessen, zum Beispiel bei entzündlichem Rheuma (akuter Schub) und bei akuten Erkrankungen, die mit körpereigener Wärmeentwicklung (lokale Entzündung, Rötung, Überwärmung, Fieber) einhergehen.

Die Wärmeauflage 10 sollte derart am Körper der Person 28 positioniert sein, das kontraktierte Muskeln welche unter der Haut der Person 28 liegen, stimuliert d.h. mit Wärmeenergie der Wärmeauflage 10 versorgt werden können. Durch die Wärmeauflage 10 kann Energie bzw. Wärmeenergie an den Körper der Person 28 bzw. an kontraktierte Muskeln abgegeben werden. Je präziserer die Wärmeauflage 10 über kontraktierten bzw. verspannten Muskeln angeordnet sind, desto präziser kann die Energie an den kontraktierten Muskel abgegeben werden. Somit können Streuverluste durch unnötige Energieabgabe an Orten, welche keine verspannten Muskeln aufweisen, reduziert werden und damit kann der gesamt Energieverbrauch durch effizienten Einsatz der Energie reduziert werden. Damit halten die Batterien länger. Vorzugsweise liegt die Wärmauflage 10 möglichst nahe oder direkt am Körper an um die Energie möglichst verlustfrei an den Körper zu übertragen.

Die Wärmeauflage 10 kann im Bereich des Nackens oder der Wirbelsäule angeordnet werden. Im Bereich des Nackens und/oder der Wirbelsäule treten häufig Verspannungen bzw. Verkrampfungen oder anhaltende Kontraktionen der Muskulatur auf. Durch die Wärmeauflagen 10 wird den betroffenen Stellen bzw. Muskeln thermische Energie zugeführt, welche diese entkrampft bzw. entspannt. Es kann damit eine Dekontraktion des Muskels im Körper der Person erfolgen. Die an den Körper der Person abgegebene Energie, kann eine Veränderung der Kontraktion der Muskulatur unter der Haut der Person erzeugt. Durch die Anordnung in diesem Bereich kann die Energie mit wenig Streuverlust an die betroffenen Stellen mit Verspannungen abgegeben werden.

Die Wärmeauflagen 10 können auch an Körperstellen angeordnet werden, um einen Rückschluss über die Körperhaltung der Person 28 zu ermöglichen, bspw. an den Schultern oder an der Wirbelsäule bzw. in der Nähe der Schulter oder der Wirbelsäule. Selbstverständlich können auch je im Schulterbereich und im Rückenbereich mindestens ein Körperhaltungssensor angeordnet sein. Der Bereich des Nackens, der Schulter und die Wirbelsäule eigenen sich besonders zur Erfassung der Körperhaltung der Person. Vorzugsweise liegen die Körperhaltungssensoren möglichst nahe oder direkt am Körper an um einen Messfehler bei der Erfassung der Körperhaltungsdaten möglichst klein zu halten bzw. um Messfehler zu vermeiden.

Die Wärmeauflagen 10 können auch für verschiedene Wärme-Therapie-Szenarien eingesetzt werden (Trigger-Point-Behandlung). Dabei wird wärme an Triggerpunkte am Körper abgeben. Der oder die Signalverarbeiter können nach einem vorgegebenen Muster Steuersignale erzeugen zum Steuern der Energieabgabe von der Energiequelle an das Heizelement in verschiedenen Wärmeauflagen 10. Bei der Behandlung kann die Wärmeauflage 10 kurzzeitig erwärmt werden. Durch die Anordnung der Wärmeauflagen 10 und das aufeinander folgende Aufwärmen der Wärmeauflagen 10 kann auf dem Körper der Person 28 bspw. eine Art thermische Wellenbewegung erzeugt werden. Dies bedeutet, dass bspw. mehrere, mehr oder weniger in einer Linie liegende Wärmeauflagen 10 nacheinander eingeschaltet werden. Durch das aufeinanderfolgende einschalten bzw. aufwärmen der Wärmeauflagen 10, wird eine Art Wellenbewegung auf dem Körper der Person 28 erzeugt bzw. das aufeinander abgestimmte Aufwärmen der Wärmeauflagen 10 erzeugt für die Person 28 den Eindruck einer sich von einem Punkt fortsetzenden bzw. ausbreitenden Wärmewelle. Durch diese Wellenförmige wärme Abgabe an den Körper kann eine besonders wirkungsvolle Entspannung der Muskulatur erreicht werden. Eine erste Wärmeauflage 10 kann bspw. zwischen 5 min und 2 h vorzugsweise zwischen 20 min und 1 h mit elektrischer Energie versorgt und dadurch warm gehalten werden. Danach wird eine zweite Wärmeauflage 10 für eine ähnliche Zeitdauer mit elektrischer Energie versorgt und dadurch warmgehalten. Danach kann erneut die erste oder eine dritter oder weitere Wärmeauflage 10 erwärmt und warmgehalten werden.

Eine Heizauflage 14 kann kontinuierlich mit einer vorgegebenen Energie, bspw. einer vorgegebenen Spannung (z.B. 5 Volt) oder einem vorgegebenen Strom versorgt werden. Die Heizauflage 14 kann zur Steuerung der Wärme mit einer geringeren Spannung (bspw. 3 V) oder höheren Spannung (z.B. 7 V) oder Strom versorgt werden. Es ist auch möglich, die Heizauflage 14 mit einer kontantem Strom oder Spannung und durch verändern der Ein-Ausschalt-Zeit (Puls-Paus-Verhältnis) in ihrer Erwärmung zu steuern. So kann die Heizauflage 12 z.B. während 2 Sekunden mit elektrischer Energie versorgt und während 1 Sekunde nicht mit elektrischer Energie versorgt werden. Über das Verhältnis der Zeitdauer zwischen Energiezufuhr und nicht Zufuhr, kann die Erwärmung der Heizauflage gesteuert werden. Die Zeitdauer für ein optimales Puls-Paus-Verhältnis hängt von der thermischen Kapazität des Heizelementes ab und kann zwischen Millisekunden und ein paar wenigen Minute liegen.

Dabei kann autonom und intelligent die Wärme auf die einzelnen Wärmeauflagen 10 bzw. Wärme-Patches untereinander geregelt werden. Dies bedeutet jede Wärmeauflage 10 kann ihre Temperatur selbst steuern oder Steuerbefehle empfangen. Die Wärmeauflage 10 kann Daten über ihre Sensoren und/oder ihren Betriebszustand bspw. weiteren Wärmeauflagen 10 mitteilen oder Daten von diesen erhalten. Durch künstliche Intelligenz kann eine intelligente Verteilung von Wärme erzeugt werden. Es kann eine Auswahl verschiedener Wärme-Therapie-Szenarien bereitgestellt werden. Durch Software und/oder künstliche Intelligenz sind beliebige Erweiterungen und Regulierungen möglich.

Abhängig von der Körperhaltung bzw. der Körperfehlhaltung kann die Energie bzw. die Wärme zu einem bestimmten Zeitpunkt ungleich bzw. intelligent verteilt werden, so dass die Wärmeauflagen 10 gleichzeitig mit unterschiedlichen Energiemengen versorgt werden. Die Energie kann auch zeitabhängig unterschiedlich bzw. intelligent verteilt werden, so dass die Wärmeauflagen 10 nacheinander mit Energie versorgt werden. Somit können Schmerzen gelindert und Verspannungen gelöst werden. Der Signalverarbeiter kann dabei ausgebildet sein, eine Körperfehlhaltung der Person auf Basis der Körperhaltungsdaten zu erkennen und die Abgabe des elektrischen Stroms an mindestens eine der Wärmeauflagen zu steuern. Dabei könne Körperdaten bzw. Körperhaltungsdaten, durch den mindestens einen Körperhaltungssensor erzeug werden. Diese Körperdaten können dem Signalverarbeiter übertragen werden. Der Signalverarbeiter kann abhängig von den Körperdaten das Steuersignal, mittels dessen die Energieabgabe von der Energiequelle an das Heizelement steuerbar ist erzeugen.

Figur 6 zeigt ein System 30 zur Abgabe von Wärmeenergie an den Körper einer Person mit verschiedenen Wärmeauflagen 10. Das System kann mindestens zwei Wärmeauflagen 10 mit einer Funkeinheit umfassen, wobei die Wärmeauflagen 10 ausgebildet sind Daten zu senden und/oder Daten zu empfangen. Die Wärmeauflagen 10 könnten auch über ein Kabel bspw. untereinander und/oder mit einer zentralen Energiequelle und/oder einem Signalverarbeiter verbunden sein. Die Wärmeauflagen 10 können zum Datenaustausch auch miteinander bspw. über eine Funkverbindung 32 verbunden sein. Die Daten können Körperhaltungsdaten, Temperaturdaten, Daten zur Steuerung der Energiequelle, Signalverarbeitungsdaten, Sensordaten, Steuerdaten, Betriebsdaten oder andere beliebige Daten sein. Die Daten können dabei von einer ersten Wärmeauflage 10 zu einer zweiten Wärmeauflage 10 gesendet werden. Somit können die Wärmeauflagen 10 Daten untereinander bzw. zwischen einander austauschen und damit ein System 30 bzw. ein Netzwerk bilden. Die Verbindung kann bspw. über eine Funkverbindung 32, über ein Piconet wie Bluetooth, RF24, RF32, ANT oder NFC (Near Field Communication) erfolgen. Die Wärmeauflagen 10 können auch über elektrische Leitungen miteinander oder zu einer zentrale Steuerung oder Energieversorgung verbunden sein.

Die Wärmeauflagen 10 können allgemein, wie in der Figur gezeigt, vielfältige Formen aufweisen. Insbesondere können die Wärmeauflagen 10 rund, oval, viereckig oder eine andere vorzugsweise an den Verlauf der Muskulatur, welche stimuliert werden sollen, angepasste Form aufweisen.

Figur 7 zeigt ein System 30 zur Abgabe von Wärmeenergie an den Körper einer Person mit einem Mobilgerät 34. Das System 30 kann mindestens eine erste Wärmeauflage 10, und mindestens eine zweite Wärmeauflage 10 und ein Mobilgerät 34 umfassen. Die erste und zweite Wärmeauflage 10 und das Mobilgerät 34 sind geeignet Daten untereinander auszutauschen. Jede der gezeigten Wärmeauflage 10 kann ausgebildet sein Daten an ein Mobilgerät 34 zu senden und/oder von einem Mobilgerät 34 zu empfangen. Die Wärmeauflage 10 kann zum Datenaustausch durch die Funkeinheit mit einem Mobiltelefon 34 verbunden sein. Die Daten können wiederum Körperhaltungsdaten, Temperaturdaten, Daten zur Steuerung der Energiequelle, Signalverarbeitungsdaten, Sensordaten, Steuerdaten, Betriebsdaten oder andere beliebige Daten sein. Die Daten können dabei von der Wärmeauflage 10 zu dem Mobiltelefon 34 oder zu einer anderen Wärmeauflage 10 gesendet werden. Somit können die Wärmeauflagen 10 Daten zwischen dem Mobiltelefon 34 oder weiteren Wärmeauflagen 10 austauschen und damit ein System 30 bzw. ein Netzwerk bilden. Die Verbindung kann bspw. über eine Funkverbindung 32, über ein Piconet wie Bluetooth, RF24, RF32, ANT oder NFC (Near Field Communication) erfolgen. Das Mobilgerät 34 kann ein Mobiltelefon bzw. ein Smartphone oder ein Tablet sein oder ein speziell für die Verbesserung der Körperhaltung ausgebildetes mobiles Gerät sein. Die Wärmeauflagen 10 können auch über elektrische Leitungen bspw. mit dem Mobilgerät 34 oder einer Energieversorgung verbunden sein.

Durch ein Mobilgerät 34 wie z.B. ein Smartphone oder ein Tablet können bspw. über eine Software-Applikation (App) Daten empfangen, ausgewertet bzw. dargestellt werden. Der Signalverarbeiter 16 kann auch teilweise oder vollständig in dem Mobilgerät 34 angeordnet sein. Das Mobilgerät 34 kann einen Signalverarbeiter 16 enthalten, welcher die Körperhaltungsdaten, über eine Schnittstelle des Mobilgerätes 34, bspw. von dem Körperhaltungssensor empfängt. Das Mobilgerät 34 kann an einer Schnittstelle aber auch verarbeitete Daten vom Signalverarbeiter 16 erhalten und diese beispielsweise darstellen oder speichern. Das Mobilgerät 34 kann an seiner Schnittstelle aber auch bspw. teilweise durch den Signalverarbeiter 16 bearbeite Körperhaltungsdaten empfangen und diese zusätzlich verarbeiten.

Durch eine App können der Person Hinweise zur Verbesserung der Körperhaltung gegeben werden.

Durch die App kann die Person auch bspw. einzelne Wärmeauflagen 10 manuell ansteuern und mit Energie versorgen oder Bereiche am Körper kennzeichnen, welche sie als Verspannt wahrnimmt und mit Wärmeenergie versorgen haben will, um die Bereiche zu entspannen. Oder einen intelligenten, autonomen Wärmemodus einstellen. Eine intelligente Steuerung kann dabei aufgrund gespeicherter Daten und Algorithmen die Heizauflage 12 der Wärmeauflage 10 mit Energie versorgen bzw. erwärmen. Dabei ist es auch möglich, dass der Benutzer z.B. Wärmemuster selber definieren kann, bspw. durch zeichnen oder verändern von Linien in Grafiken. Auch kann das System 30 zuerst auf vorbestehende Körperfehlhaltung bspw. durch angeborene Fehlhaltung oder Erkrankung kalibriert werden. Im weiteren können dann Abweichungen zu dieser vorbestehenden Körperfehlhaltung erfasst bzw. behandelt werden.

Die App kann der Person bzw. dem Benutzer auch eine Empfehlung zur Verbesserung der Körperhaltung ausgeben, bspw. indem die App der Person grafisch bzw. durch Text anzeigt, wie die Körperhaltung zu verbessern ist. Die App kann bspw. auch Vorschläge zu Turn- bzw. Gymnastikübungen, zur Verbesserung der Körperhaltung ausgeben. Die App kann der Person auch Informationen aus dem Internet zugänglich machen, welche bspw. der Person helfen die Körperhaltung zu verbessern. Ferner kann die App auch bspw. durch einen Film Informationen über die Fehlhaltung der Person bzw. Verbesserungsvorschläge an die Person ausgeben.

Die Verbesserung der Körperhaltung der Person kann durch bewusste Veränderung der Körperhaltung der Person erfolgen, z.B. in dem die Person durch Vibration oder elektrische Impulse einer Wärmeauflage 10 oder durch eine Anzeige auf einem Mobilgerät 34 über eine Körperfehlhaltung informiert wird. Die Verbesserung der Körperhaltung bzw. die Korrektur der Körperfehlhaltung kann aber auch für die Person unbewusst erfolgen, indem einzelne Muskeln oder Muskelgruppen durch die Zufuhr von Energie aus der Wärmeauflage 10 entspannt werden.

In Figur 8a sind die Umrisse eines Oberkörpers einer Person 28 bspw. auf einem Display eines Mobilgeräts 34 wie bspw. einem Smartphone dargestellt. In der Silhouette auf dem Display können an dem Körper angeordnete Wärmeauflagen 10 und/oder Körperhaltungssensoren 24 dargestellt sein. Die Person welche das System 30 zur Abgabe der Wärmeenergie an den Körper einer Person benutzt, kann als Benutzer bezeichnet werden. Der Benutzer kann bspw. die Anordnung der Körperhaltungssensoren 24 oder der Wärmeauflagen 10 auf dem Körper eingeben, prüfen oder verändern. Es ist ferner möglich, dass die Person 28 die Zufuhr der elektrischen Energie bzw. die Intensität mit welcher thermische Energie an den Körper abgegeben wird z.B. über den Touchscreen des Mobilgerätes 34 beeinflusst bzw. steuert. Die Beeinflussung der Energieabgabe ist bspw. mit einem PlusZeichen und einem Minus-Zeichen auf dem Display des Mobilgerätes 34 dargestellt. Durch berühren des Plus-Zeichens kann der Benutzern die Energieabgabe bspw. erhöhen. Durch berühren des Minus-Zeichens kann der Benutzern die Energieabgabe bspw. verringern.

Figur 8b zeigt den Oberkörper einer Person 28 auf dem Display eines Mobilgeräts 34 wie bspw. einem Smartphone. Auf dem Display kann dem Benutzer z.B. seine von dem System erkannte Körperfehlhaltung angezeigt werden. Auf dem Display kann dem Benutzer ferner angezeigt werden, wie er seine Körperhaltung verbessern kann. Die Ausgabe der Verbesserungsvorschläge kann dabei mit Symbolen ergänzt oder animiert erfolgen.

Figur 8c zeigt erneut den Oberkörper einer Person 28 auf dem Display eines Mobilgeräts 34 wie bspw. einem Smartphone. Das Smartphone kann als Mobilgerät 34 Vorschläge für die Verbesserung der Körperhaltung an den Benutzer ausgeben. Die Vorschläge können z.B. Bewegungs- oder Turnübungen umfassen, welche der Benutzer ausführen soll. Die Vorschläge können in einer vorgegebenen zeitlichen Regelmässigkeit dem Benutzer mitgeteilt werden, so dass der Benutzer bspw. einmal täglich zu einer bestimmten Zeit daran erinnert wird bzw. aufgefordert wird Bewegungs- oder Turnübungen zu machen. Die Übungen helfen dem Benutzer z.B. verspannte Muskel zu entspannen oder Muskel zu kräftigen und dadurch die Körperhaltung zu verbessern. Die Übungen können als aktive Massnahmen bezeichnet werden. Die Übungen können personalisiert auf einen bestimmten Benutzer oder auf eine bestimmte Fehlhaltung abgestimmt, automatisiert zusammengestellt und ausgegeben werden. Die regelmässig Benachrichtigung motiviert zu mehr Bewegung. Dies kann chronischen Verspannungen und Schmerzen entgegenwirken.

Die Kombination von aktiven sowie passiven Massnahmen führen zu einer gesunden und richtigen Körperhaltung. Konstant konzentrierte Wärme löst Verspannungen damit der Körper wieder in die natürliche Haltung zurückfinden kann. Somit entstehen keine Langfristigen negativen Folgen.

In Ausführungsbeispielen kann die Heizauflage einen Allergiefreundlichen bzw. Hautfreundlichen gelartigen Klebstoff umfassen. Dadurch besteht weniger Gefahr einer allergischen Reaktion der Haut (Allergiefreundlich). Ferner kann die elektrische Energiequelle aufladbar sein und können somit nachhaltiger als ein herkömmliches Wärmepflaster sein. Da bei der Wärmeauflage die Wärme durch elektrische Energie und nicht wie bei einem Wärmepflaster durch eine chemische Reaktion erzeugt wird, besteht bei der vorliegenden Wärmeauflage weniger die Gefahr einer allergischen Reaktion auf die chemische Stoffe.

Die Wärmeauflage 10 kann auch als Wärmepatch, Wärmepad, Wärmeunterlage, Wärmepflaster, Wärmematte oder Wärmekissen bezeichnet werden. Die Wärmeauflage kann zur lokalen positionsbestimmten Abgabe von thermischer Energie an die Haut bestimmt sein. Die Heizauflage kann auch als Heizpflaster oder Heizpatch bezeichnet werden. Die Steuereinheit kann auch als Steuerpatch bezeichnet werden.

In Ausführungsbeispielen kann die Wärmeauflage auch ausgebildet sein Energie in verschiedenen Formen an den Körper abzugeben. Die Abgabe verschiedener Energieformen an den Körper kann dabei zeitgleich oder sequentiell erfolgen. So kann z.B. in einer ersten Phase thermische Energie durch die Wärmeauflage 10 an den Körper der Person abgegeben werden. In einer zweiten Phase, kann dieselbe Wärmeauflage bspw. mechanische oder elektrische Energie an den Körper der Person abgeben. Durch die Stimulierung mit verschiedenen Energieformen (mechanisch, thermisch, elektrisch) kann teilweise eine schnellere und nachhaltigere Entspannung des Muskels erzielt werden.

Ausführungsbeispiele zeigen eine Wärmeauflage zur Abgabe von Wärmeenergie an den Körper einer Person. Die Wärmeauflage umfasst eine Heizauflage mit einer Klebefläche, zum ankleben der Heizauflage an der Hautoberfläche einer Person, wobei die Klebefläche an einer Unterseite der Heizauflage vorgesehen ist und die Heizauflage ausgebildet ist, elektrische Energie in Wärmeenergie umzuwandeln. Eine Steuereinheit an welchem ein Signalverarbeiter angeordnet sein kann, wobei der Signalverarbeiter ausgebildet sein kann ein Signal zur Steuerung einer elektrischen Energiequelle zu erzeugen. Ein erstes elektrisches Verbindungselement zur Übertragung eines elektrischen Stroms von der Steuereinheit auf ein dazu komplementäre zweites elektrisches Verbindungselement, welches an der Heizauflage ausgebildet ist. Mindestens ein erstes mechanisches Verbindungselement, welches an der Steuereinheit angeordnet ist und ein komplementäres zweites mechanisches Verbindungselement, welches an einer der Klebefläche gegenüberliegenden Oberseite an der Heizauflage angeordnet ist, sodass die Steuereinheit mit der Heizauflage verbindbar ist.

Gewisse Aspekte wurden im Zusammenhang mit einer Vorrichtung beschrieben, gleichwohl können diese Aspekte auch eine Beschreibung des entsprechenden Verfahrens darstellen, sodass ein Block oder ein Element einer Vorrichtung auch als ein entsprechender Verfahrensschritt oder als ein Merkmal eines Verfahrensschrittes verstanden werden kann.

Entsprechend dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar. Teile oder alle der Verfahrensschritte können unter Verwendung eines Hardware-Apparats, wie zum Beispiel einem Smartphone, einem Mikroprozessor, einen programmierbaren Computer oder eine elektronische Schaltung ausgeführt werden. Bei einigen Ausführungsbeispielen können einige oder mehrere der wichtigsten Verfahrensschritte durch einen solchen Hardware-Apparat ausgeführt werden.

Abhängig von Umsetzungsanforderungen können Ausführungsbeispiele der Erfindung in Hardware oder in Software umgesetzt sein. Die Umsetzung kann unter Verwendung eines digitalen Speichermediums, beispielsweise eines Memorysticks, einer Speicherkarte, einer DVD, einer Blu-ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einem programmierbaren Computersystem derart zusammenwirken können oder zusammenwirken, dass das jeweilige Verfahren durchgeführt wird. Somit kann das digitale Speichermedium computerlesbar sein.

Somit können Ausführungsbeispiele gemäss der Erfindung einen Datenträger umfassen, der elektronisch lesbare Steuersignale aufweist, welche in der Lage sind, mit einem programmierbaren Computersystem so zusammenzuwirken, dass eines der hierin beschriebenen Verfahren durchgeführt werden kann.

Ausführungsbeispiele der vorliegenden Erfindung können als Computerprogramm mit einem Programmcode umgesetzt sein, wobei der Programmcode dahin gehend wirksam ist, eines der Verfahren durchzuführen, wenn das Computerprogramm auf einem Computer abläuft. Der Programmcode kann beispielsweise auch auf einem maschinenlesbaren Träger gespeichert sein. Andere Ausführungsbeispiele umfassen das Computerprogramm zum Durchführen eines der hierin beschriebenen Verfahren, wobei das Computerprogramm auf einem maschinenlesbaren Träger gespeichert ist.

Ein Ausführungsbeispiel des erfindungsgemäßen Verfahrens ist somit anders Ausgedrückt, ein Computerprogramm, das einen Programmcode zum Durchführen eines der hierin beschriebenen Verfahren aufweist, wenn das Computerprogramm auf einem Computer abläuft. Der Datenträger, das digitale Speichermedium oder das aufgezeichnete Medium sind üblicherweise greifbar bzw. nicht-flüchtig. Ferner ist ein weiteres Ausführungsbeispiel der erfindungsgemäßen Verfahren ein Datenträger (oder ein digitales Speichermedium oder ein computerlesbares Medium), auf dem das Computerprogramm zum Durchführen eines der hierin beschriebenen Verfahren aufgezeichnet ist.

Ein weiteres Ausführungsbeispiel des erfindungsgemäßen Verfahrens ist ein Datenstrom oder eine Sequenz von Signalen, der bzw. die das Computerprogramm zum Durchführen eines der hierin beschriebenen Verfahren darstellt bzw. darstellen. Der Datenstrom oder die Sequenz von Signalen kann bzw. können beispielsweise dahin gehend konfiguriert sein, über eine Datenkommunikationsverbindung, beispielsweise über das Internet, transferiert zu werden. Ein weiteres Ausführungsbeispiel umfasst eine Verarbeitungseinrichtung, beispielsweise einen Computer, ein Tablet, ein Smartphone oder ein programmierbares Logikbauelement, die dahin gehend konfiguriert oder angepasst ist, eines der hierin beschriebenen Verfahren durchzuführen. Ein weiteres Ausführungsbeispiel umfasst einen Computer, auf dem das Computerprogramm zum Durchführen eines der hierin beschriebenen Verfahren installiert ist.

Ein weiteres Ausführungsbeispiel gemäß der Erfindung umfasst eine Vorrichtung oder ein System, die bzw. das dazu konfiguriert ist, ein Computerprogramm zur Durchführung zumindest eines der hier beschriebenen Verfahren an einen Empfänger zu übertragen. Die Übertragung kann beispielsweise elektronisch oder optisch erfolgen. Der Empfänger kann beispielsweise ein Computer, ein Mobilgerät, ein Speichergerät oder eine ähnliche Vorrichtung sein. Die Vorrichtung oder das System kann beispielsweise einen Datei-Server zur Übertragung des Computerprogramms an den Empfänger umfassen. Bei Ausführungsbeispielen kann ein programmierbares Logikbauelement (beispielsweise ein feldprogrammierbares Gatterarray, ein FPGA) dazu verwendet werden, manche oder alle Funktionalitäten der hierin beschriebenen Verfahren durchzuführen. Bei manchen Ausführungsbeispielen kann ein feldprogrammierbares Gatterarray mit einem Mikroprozessor zusammenwirken, um eines der hierin beschriebenen Verfahren durchzuführen. Allgemein werden die Verfahren bei einigen Ausführungsbeispielen mit einer beliebigen Hardwarevorrichtung durchgeführt. Diese kann eine universell einsetzbare Hardware wie ein Computerprozessor (CPU) oder für das Verfahren spezifische Hardware, wie beispielsweise ein ASIC, sein.

Die oben beschriebenen Ausführungsbeispiele stellen lediglich eine Veranschaulichung der Prinzipien der vorliegenden Erfindung dar. Modifikationen und Veränderungen der hierin beschriebenen Anordnungen und Einzelheiten können anderen Fachleuten offenkundig sein. Deshalb soll die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sein.

Bezugszeichenliste

| 10 | Wärmeauflage |
|----|----|
| 12 | Heizauflage |
| 121 | Unterseite der Heizauflage |
| 1210 | Klebefläche |
| 122 | Oberseite der Heizauflage |
| 14 | Steuereinheit |
| 16 | Signalverarbeiter |
| 18 | elektrischer Verbinder |
| 18a | erstes elektrisches Verbindungselement (an der Steuereinheit) |
| 18b | zweites elektrisches Verbindungselement (an der Heizauflage) |
| 20 | mechanischer Verbinder |
| 20a | erstes mechanisches Verbindungselement (an der Steuereinheit) |

| | |
|---|---|
| 20b | zweites mechanisches Verbindungselement (an der Heizauflage) |
| 22 | elektrische Energiequelle |
| 24 | Körperhaltungssensor |
| 241 | Biegesensor |
| 26 | Heizelement |
| 28 | Person |
| 30 | System, Netzwerk |
| 32 | Funkverbindung |
| 34 | Mobilgerät |
| 36 | Funkeinheit |
| 38 | Temperatursensor |
| 40 | Elektroden |
| 42 | Vibrationserzeuger |

## Patentansprüche

1. Wärmeauflage (10) zur Abgabe von Wärmeenergie an den Körper einer Person (28), umfassend:

   eine mit dem Körper der Person (28) verbindbare Heizauflage (12), die eine Oberseite (122) und eine Unterseite (121) aufweist und an deren Unterseite (121) eine Klebefläche (1210), zum Ankleben der Heizauflage (12) an der Hautoberfläche der Person (28) vorgesehen ist, und die wenigstens ein Heizelement (26) umfasst, durch das von einer elektrischen Energiequelle (22) abgegebene elektrische Energie in Wärmeenergie wandelbar ist,
   eine Steuereinheit (14), die zur festen oder lösbaren Aufnahme der Energiequelle (22) vorgesehen ist, die einen Signalverarbeiter (16) aufweist der zur Abgabe eines Steuersignals vorgesehen ist, mittels dessen die Energieabgabe von der Energiequelle (22) an das Heizelement (26) steuerbar ist;
   wobei die Heizauflage (12) und die Steuereinheit (14) durch wenigstens einen elektrischen Verbinder (18), über den die elektrische Energie zum Heizelement (26) übertragbar ist, und wenigstens einen mechanischen Verbinder (20), elektrisch und mechanisch miteinander verbindbar sind.

2. Wärmeauflage (10) nach Anspruch 1, wobei der elektrische Verbinder (18) ein mit der Steuereinheit (14) verbundenes erstes elektrisches Verbindungselement (18a) und ein mit der Heizauflage (12) verbundenes zweites Verbindungselement (18b) aufweist, und dass der mechanische Verbinder (20) ein mit der Steuereinheit (14) verbundenes erstes mechanisches Verbindungselement (20a) und ein mit der Heizauflage (12) verbundenes zweites mechanisches Verbindungselement (20b) aufweist, und dass das erste und zweite mechanische Verbindungselement (20a; 20b) das dazu korrespondierende erste bzw. zweite elektrische Verbindungselement (18a; 18b) umschliesst..

3. Wärmeauflage (10) nach Anspruch 1 oder 2, wobei mindestens drei mechanische Verbindungselemente (20a) an der Steuereinheit (14) und an gleicher Stelle gegenüberliegend an der Heizauflage (12) drei komplementäre mechanische Verbindungselemente (20b) zum Verbinden der Heizauflage (12) mit der Steuereinheit (14) ausgebildet sind.

4. Wärmeauflage (10) nach einem der Ansprüche 1 bis 3, wobei die elektrische Energiequelle (22) und das Heizelement (26) ausgebildet sind, die Heizauflage (12) in weniger als 1 min vorzugsweise in weniger als 20 sek., besonders bevorzugt in weniger als 5 sek. um 20° C zu erwärmen.

5. Wärmeauflage (10) nach einem der Ansprüche 1 bis 4, wobei die Wärmeauflage (10) mindestens einen Körperhaltungssensor (24, 241) umfasst und der Körperhaltungssensor (24, 241) ausgebildet ist, die Körperhaltung und/oder die Bewegung der Person (28) zumindest in Teilen zu erfassen und Körperhaltungsdaten zu erzeugen und der Signalverarbeiter (16) ist ausgebildet, abhängig von den Körperhaltungsdaten das Steuersignal zur Steuerung der elektrischen Energiequelle (22) zu erzeugen.

6. Wärmeauflage (10) nach einem der Ansprüche 1 bis 5, wobei die Wärmeauflage (10) eine Funkeinheit (36) zum Senden und/oder Empfangen von Daten umfasst.

7. Wärmeauflage (10) nach einem der Ansprüche 1 bis 6, wobei die Wärmeauflage (10) einen Temperatursensor (38) umfasst, welcher ausgebildet ist Temperaturdaten zu erzeugen und der Signalverarbeiter (16) ist ausgebildet, abhängig von den Temperaturdaten das Steuersignal zur Steuerung der elektrischen Energiequelle (22) zu erzeugen.

8. Wärmeauflage (10) nach einem der Ansprüche 1 bis 7, wobei an der Unterseite der Heizauflage (12) Elektroden (40) angeordnet sind und der Signalverarbeiter (16) ausgebildet ist, durch Elektromyografie die Muskelspannung und/oder durch Messen des Leitwertes den Hautwiderstand zu bestimmen.

9. Wärmeauflage (10) nach einem der Ansprüche 1 bis 8, wobei die Klebefläche (121) der Heizauflage (12) einen Klebstoff, vorzugsweise einen Haftklebstoff umfasst.

10. Wärmeauflage (10) nach einem der Ansprüche 1 bis 9, wobei der Klebstoff gelförmig ausgebildet ist.

**11.** Wärmeauflage (10) nach einem der Ansprüche 1 bis 10, wobei die Heizauflage (12) ein Wegwerfelement ist und die Steuereinheit (14) ein wiederverwendbares Element ist.

**12.** Wärmeauflage (10) nach einem der Ansprüche 1 bis 11, wobei die Wärmeauflage (10) einen Vibrationserzeuger (42) umfasst, welcher ausgebildet ist, von der elektrischen Energiequelle (22) abgegebene elektrische Energie in mechanische Energie umzuwandeln, die an den Körper der Person (28) abgebbar ist.

**13.** System (30) zur Abgabe von Wärmeenergie an den Körper einer Person (28) zur Entspannung der Körpermuskulatur umfassend:

mindestens eine erste Wärmeauflage (10) gemäss einem der Ansprüche 1 bis 12, und mindestens eine zweite Wärmeauflage (10) gemäss einem der Ansprüche 1 bis 12 und/oder ein Mobilgerät (34), die geeignet sind, Daten mit der ersten Wärmeauflage (10) auszutauschen.

**14.** Verfahren zur Steuerung einer Wärmeauflage (10) gemäss einem der Ansprüche 1 bis 12, die zur Abgabe von Wärmeenergie an den Körper einer Person (28) vorgesehen ist und die eine Steuereinheit (14) und eine mit dem Körper der Person (28) verbindbare und mit einem Heizelement (26) versehene Heizauflage (12) umfasst, die elektrisch und mechanisch miteinander verbunden werden, wobei mittels eines Signalverarbeiters (16) ein Steuersignal erzeugt wird, mittels dessen die Energieabgabe von einer Energiequelle (22) an das Heizelement (26) steuerbar ist.

**15.** Verfahren nach Anspruch 14, wobei mittels der Körperhaltungssensoren (24) die Körperhaltung der Person (28) gemessen und in dem Signalverarbeiter (16) ermittelt und daraus das Steuersignal berechnet wird, um die Wärmeabgabe derart zu steuern, dass die Körperhaltung korrigiert wird.

**16.** Computerprogramm mit einem Programmcode zur Durchführung des Verfahrens nach Anspruch 14 oder 15, wenn das Computerprogramm auf einem Computer oder Prozessor läuft.

Fig. 1

Fig. 2

Fig. 3a

Fig. 3b

10

42  36  16  24  22  14

18a; 20a

12  18b; 20b  26  38

40  241  241  40

Fig. 4

28  28

10  10

Fig. 5a  Fig. 5b

30

Fig. 6

30

Fig. 7

Fig. 8a

Fig. 8b

Fig. 8c

Europäisches
Patentamt

European
Patent Office

Office européen
des brevets

# EUROPÄISCHER RECHERCHENBERICHT

**Nummer der Anmeldung**

EP 18 15 4286

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2016/331959 A1 (HSIEH CHIN-CHIH [TW]) 17. November 2016 (2016-11-17) | 1-4, 9-11,14, 16 | INV. A61F7/00 A61F7/02 |
| Y | * Zusammenfassung; Abbildungen 1-4 * * Absatz [0018] - Absatz [0030] * ----- | 5-8,12, 13,15 | |
| Y | KR 2015 0083559 A (GWEAN GI REANG [KR]; MOTODESIGN CONSULTING INC [KR]) 20. Juli 2015 (2015-07-20) * Zusammenfassung; Abbildungen 2, 3 * * Absatz [0055] * ----- | 5,6,13, 15 | |
| Y | US 8 658 943 B1 (LARSEN WALTER G [US] ET AL) 25. Februar 2014 (2014-02-25) * Zusammenfassung; Abbildung 2A * ----- | 7 | |
| Y | DE 10 2012 203880 A1 (BOSCH GMBH ROBERT [DE]) 19. September 2013 (2013-09-19) * Zusammenfassung * * Absatz [0002] * ----- | 8 | |
| Y | US 2015/297445 A1 (GORDON CHARLES R [US] ET AL) 22. Oktober 2015 (2015-10-22) * Zusammenfassung * * Absatz [0039] * ----- | 12 | **RECHERCHIERTE SACHGEBIETE (IPC)** A61F |

Der vorliegende Recherchenbericht wurde für alle Patentansprüche erstellt

| Recherchenort | Abschlußdatum der Recherche | Prüfer |
|---|---|---|
| München | 14. Juni 2018 | Wetzig, Thomas |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument

..................................................................................
& : Mitglied der gleichen Patentfamilie, übereinstimmendes
   Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 18 15 4286

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-06-2018

| Im Recherchenbericht angeführtes Patentdokument | Datum der Veröffentlichung | Mitglied(er) der Patentfamilie | Datum der Veröffentlichung |
|---|---|---|---|
| US 2016331959 A1 | 17-11-2016 | KEINE | |
| KR 20150083559 A | 20-07-2015 | KEINE | |
| US 8658943 B1 | 25-02-2014 | US 8658943 B1<br>US 2014200637 A1<br>WO 2014113271 A2 | 25-02-2014<br>17-07-2014<br>24-07-2014 |
| DE 102012203880 A1 | 19-09-2013 | DE 102012203880 A1<br>US 2013245546 A1 | 19-09-2013<br>19-09-2013 |
| US 2015297445 A1 | 22-10-2015 | KEINE | |

EPO FORM P0461

Für nähere Einzelheiten zu diesem Anhang : siehe Amtsblatt des Europäischen Patentamts, Nr.12/82